# EUROPEAN PATENT APPLICATION

(11) **EP 3 203 233 A2**
(43) Date of publication of application: **09.08.2017**
(21) Application number: 16204570.2
(22) Date of filing: 28.10.2009
(51) Int. Cl.: G01N 33/53, G01N 33/569, G01N 33/574, G01N 33/68

(54) **METHODS FOR USING ANTIBODIES AND ANALOGS THEREOF**

(30) Priority: 29.10.2008 US 197601 P; 21.09.2009 US 563330; 27.10.2009 US 606818
(62) Divisional of application: 09825245.5
(71) Applicant: ICB International, Inc., San Diego, CA 92130 (US)
(72) Inventor: BHATT, Ram, S., San Diego, CA 92130 (US); BHATT, Rishi, San Diego, CA 92130 (US)
(74) Representative: Schiweck, Weinzierl & Koch Patentanwälte Partnerschaft mbB

(57) **Abstract**

Camelid and shark single-domain heavy chain antibodies lacking the light chains, and their analogs are disclosed. Methods for using the antibodies and their analogs to detect antigens are disclosed. Methods to derivatize such antibodies and analogs to develop diagnostic assays are described. Also provided are kits, and methods of using such diagnostics assays.

## Description

### CROSS-REFERENCE TO RELATED APPLICATION

This application claims the benefit of U.S. Provisional Application 61/197,601 filed on October 29, 2008 and is a continuation-in-part of U.S. application Ser. No. 12/563,330 filed September 21, 2009 which claims the priority of U.S. Provisional Application No. 61/192,732 filed September 22, 2008, each of which is hereby incorporated by reference in its entirety.

### FIELD OF THE INVENTION

This invention relates to the use of single-domain heavy-chain only camelid and shark antibodies and their analogs without the light-chains.

### BACKGROUND OF THE INVENTION

The occurrence of various cancers and diseases usually involves pathological antigens, altered protein expression, and/or distribution [Nature, 422, 226 (2003)]. The detection of low levels of certain protein biomarkers can be extremely useful for the diagnosis, prognosis and treatment of specific cancers and other diseases for effective disease control and/or treatment.

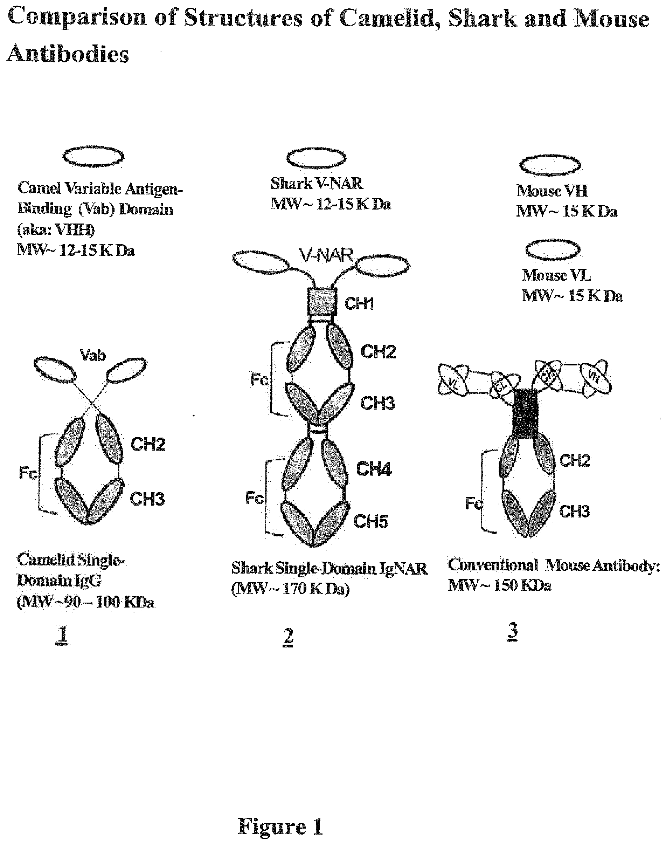

A class of naturally occurring antibodies have been identified from camelids and sharks. In addition to classical heterotetrameric antibodies, camelids and sharks also produce so called "incomplete antibodies" without the light-chains. Their structure is shown in figure 1.

Thus, two types of antibodies exist in camels, dromedaries and llamas: one a conventional hetero-tetramer having two heavy and two light chains (MW~160 K Da), and the other consisting of only two heavy chains, devoid of light chains (MW ~90 KDa) and also deprived of constant region CH1.

In addition to camelid antibodies having only two heavy chains and devoid of light chains, distinctly unconventional antibody isotype was identified in the serum of nurse sharks (Ginglymostoma cirratum) and wobbegong sharks (Orectolobus maculatus). The antibody was called the immunoglobulin (Ig) new antigen receptors (IgNARs). They are disulfide-bonded homodimers consisting of five constant domains (CNAR) and one variable domain (VNAR). There is no light chain, and the individual variable domains are independent in solution and do not appear to associate across a hydrophobic interface. Like the Vab domain of camelid antibodies, the variable antigen-binding domain , known as V-NAR, of single-domain shark antibodies is also stable by itself and has a molecular weight of about 15 KDa (Greenberg, A. S., Avila, D., Hughes, M., Hughes, A., McKinney, E. & Flajnik, M. F., Nature, 374, 168-173 (1995); Mol. Immunol. 38, 313-326, (2001); Comp. Biochem. Physiol. B., 15, 225 (1973)). There are three different types of IgNARs characterized by their time of appearance in shark development, and by their disulfide bond pattern [Diaz, M., Stanfield, R. L., Greenberg, A. S. & Flajnik, M. F., Immunogenetics 54, 501-512 (2002); Nuttall, S. D., Krishnan, U. V., Doughty, L., Pearson, K., Ryan, M. T., Hoogenraad, N. J., Hattarki, M., Carmichael, J. A., Irving, R. A. & Hudson, P. J., Eur. J. Biochem., 270, 3543-3554 (2003)].

**Relevant References: Foreign and US Patents:**

| | |
|---|---|
| US Patent Application 12563330 September 21, 2009 | Antibodies, Analogs and Uses Thereof |
| PCT/US2009/057681 September 21, 2009 | Antibodies, Analogs and Uses thereof |
| US Patent 7371849 (May, 2008) | Methods of constructing camel antibody libraries. |
| US Patent 6838254 B1 (Jan., 2005) | Production of antibodies or fragments thereof derived from heavy-chain immunoglobulins of camelidae. |
| US Patent 6765087 (July, 2004) | Immunoglobulins devoid of light chains. |
| US Patent 6005079 (Dec., 1999) | Immunoglobulins devoid of light chains. |
| US Patent 5800988 (Sept., 1998) | Immunoglobulins devoid of light chains. |
| WO/2002/048193 (June, 2002) | Camelidae Antibody Arrays. |
| EP 1264885 (Dec., 2002) | Antibody library. |
| WO/2001/090190 (Nov., 2001) | Single-domain antigen-binding antibody fragments derived from llama antibodies. |
| WO/2000/043507 (July, 2000) | Methods for producing antibody fragments. |
| EP 1024191 (August, 2000) | Production of chimeric antibodies from segment repertoires and displayed on phage. |
| WO/1999/042077 (August, 1999) | Recognition molecules interacting specifically with the active site or cleft of a target molecule. |

### Other References:

Azwai SM et al, Serology of Orthopoxvirus Camel Infection in Dromedary camels, Comp. Immun. Microbiol. Infect. Dis., 19, 65 (1996).
Kelly PJ, et al, Isolation and characterization of immunoglobulin-G and IgG Subclasses of the African elephant, Comp. Immun. Microbiol. Infect. Dis., 21, 65 (1998).
Linden Richard van der et al., Induction of immune responses and molecular cloning of the heavy-chain antibody repertoire of Lama glama, J. Immun. Methods, 240, 185 (2000).
Rivera H et al., Serological survey of viral antibodies in the Peruvian alpacas, Am. J. Vet. Res.,48, 189 (1987).
Kumar M et al., Biochemical studies on Indian Camels: Blood proteins and lipids, J. Sci. Industrial Res., 20c, 236 (1961).
Ungar-Waron H., Elias E., et al., Dromedary IgG: Prurification, characterization and quantitation in Sera of Dams and newborns, Isr. J. Vet. Med., 43 (3), 198 (1987).
Azwai SM et al., The isolation and characterization of camel immunoglobulin classes and subclasses, J. Comp. Path., 109, 187 (1993).
Hamers-Casterman C., Atarhouch T., et al., Naturally occurring antibodies devoid of light chains, Nature, 363, 446 (1993).
Zhang J, Tanha J. et al., Pentamerization of single-domain antibodies from phage librarics: a novel strategy for the rapid generation of high-avidity antibody reagents, J. Mol. Biol., 335, 49 (2004).

### SUMMARY OF THE INVENTION

The present invention relates to an ultrasensitive and ultraspecific method for the detection of antigens and useful for diagnosing human diseases using camelid and shark heavy chain only antibodies lacking light chain and their analogs.

In one aspect, the invention provides a method for detecting the presence or absence of an antigen in a sample. The method includes a) obtaining a sample suspected of having said antigen, b) detecting the presence or absence of the antigen in the sample utilizing a polypeptide in which the polypeptide comprises all or a portion of at least one variable antigen-binding (Vab) domain of camelid and/ or shark single-domain heavy chain antibodies lacking light-chains, at least ten contiguous amino acids derived from a source other than camelid and/ or shark single-domain heavy chain antibodies lacking light-chains and the polypeptide comprises at least one binding site for an antigen. The polypeptide binds specifically to the antigen and the binding is indicative of the presence of the antigen.

In another aspect, the invention provides a method for detecting the presence or absence of an antigen in a sample. The method includes a) obtaining a sample suspected of having said antigen, b) detecting the presence or absence of the antigen in the sample utilizing a composition having at least two polypeptides, in which each of the polypeptides includes all or a portion of at least one variable (Vab) domain of camelid and or shark single domain heavy chain antibody lacking light chain, all or a portion of at least one hinge region of camelid and or shark single domain heavy chain antibody lacking light chain in which at least one of the polypeptide includes at least one binding site for an antigen, and the polypeptides are linked to each other through at least one linker. The polypeptide binds specifically to the antigen and the binding is indicative of the presence or absence of the antigen. In one embodiment, at least one linker is a peptide bond. In another embodiment, at least one linker is other than a peptide bond. In one embodiment, the polypeptides of the composition include at least three, at least four, at least five or more variable antigen-binding (Vab) domains of camelid and or shark single domain heavy chain antibody. In some embodiments, the polypeptide may include one or more substitutions or deletions of the native amino acids.

In another aspect, the invention provides a method to improve the biodistribution and retention of the heavy chain only camelid and shark antibodies without light-chains and their analogs. In one embodiment, the molecular weight is greater than 15 to 17 KDa and can enter a cell or cross blood brain barrier (BBB), they are retained inside the cell to be diagnostically/therapeutically efficacious. In some embodiments, the molecular weight of the antibodies and their analogs are between ~ 30 to 60 KDa, more preferably 40 to 60 KDa, ideally ~ 55 KDa. In one embodiment, the invention encompasses the synthesis of a polypeptide with two or more variable antigen-binding domains to generate the polypeptide with a MW ~ 30 to 60 KDa, more preferably 40 to 60 KDa, ideally ~ 55 KDa. The polypeptide comprises camelid Vab domains and /or shark V-NAR domains, in which such constructions/preparations are performed either chemically and/or via recombinant DNA methods.

In another aspect, the invention provides a method for detecting an organism or a cell in a sample. The method includes a) obtaining a sample suspected of having such cell or organism, b) detecting the presence or absence of one or more antigens associated with the organism or a cell by utilizing the polypeptides or compositions of the above aspects of the invention such that the polypeptides or the compositions bind specifically to one or more antigens associated with the cell or organism and the binding is indicative of the presence or absence of a cell or organism in the sample. In some embodiments, the organism is a pathogenic organism such as bacteria or virus.

In another aspect, the invention provides a method for diagnosing an individual with one or more diseases. The method includes: a) obtaining a sample of bodily fluid from the individual; b) detecting the presence or absence of one or more biomarkers associated with the disease in which the detection comprises utilizing a polypeptide in which the polypeptide comprises all or a portion of at least one variable antigen-binding (Vab) domain of camelid and/ or shark single-domain heavy chain antibodies lacking light-chains, at least ten contiguous amino acids derived from a source other than camelid and/ or shark single-domain heavy chain antibodies lacking light-chains, the polypeptide binds specifically to at least one of said biomarkers and the binding of the polypeptide to one or more of the biomarkers is indicative of the presence of one or more biomarkers in the sample; c) identifying the individual as having the disease when one or more biomarkers are present in the individual's sample. In some embodiments, the method further includes determining the amount of one or more biomarkers in the sample and comparing the amount to reference values. An amount higher or lower than the reference value is indicative of a disease. In some embodiments, the reference values are the levels of the biomarkers in an individual without such one or more diseases.

In one embodiment, the polypeptide of the above aspects of the invention comprises at least two variable antigen-binding (Vab) domains of camelid and/or shark single-domain heavy- chain antibody lacking the light chains. In another embodiment, the polypeptide of the above aspects of the invention includes at least three, at least four or more variable (Vab) domains of camelid and shark heavy chain only antibody. In some embodiments, the polypeptide may include one or more substitutions or deletions of the native amino acids. In some embodiments, at least two variable antigen-binding (Vab) domains bind to two different antigens. In one embodiment of all of the above aspects of the invention, the polypeptide includes all or a portion of at least one hinge region of camelid and /or shark single domain heavy chain antibody lacking light chain.

In one embodiment of all of the above aspects of the invention, the polypeptide includes all or a portion of at least one camelid and or shark single domain heavy chain constant domain 2 (CH2). In one embodiment of all of the above aspects of the invention, the polypeptide includes all or a portion of at least one camelid and or shark single domain heavy chain constant domain 3 (CH3). In one embodiment of all of the above aspects of the invention, at least one amino acid at positions 37, 44, 45, and 47 of the Vab region is selected from the group consisting of serine, glutamine, tyrosine, histidine, asparagine, threonine, aspartic acid, glutamic acid, lysine and arginine. In some embodiments, the polypeptide may include one or more substitutions or deletions of the native amino acids.

In some embodiments of the above aspects of the invention, the polypeptide may include domains (such as variable domain or constant domain) from at least two different species such as camelid and shark, or two different camelid species such as llama, camel, alpaca and dromedaries. In some embodiments of the above aspects of the invention, the polypeptide may have improved cellular uptake, blood brain barrier permeability, biodistribution and retention.

In some embodiments the polypeptide of the above aspects of the invention is immobilized on a solid support prior to binding to said antigen. In some embodiments the polypeptide of the above aspect of the invention binds to the antigen to form a complex and the complex is immobilized on a solid support. In one embodiment, the immobilization is achieved by covalent attachment of the polypeptide to the solid surface through a spacer. In one embodiment, the length of the spacer is 1-100 nm in length. In one embodiment, the length of the spacer is 1-50 nm. In another embodiment, the length is 20 nm.

In some embodiments of the above aspects of the invention, the polypeptide is linked to at least one entity other than an antibody. In some embodiments, the entity can be solid support, radioisotope, enzyme, detectable label, ligand, fluorophore, biotin, digoxegenin, avidin, streptavidin, Fc region of IgGs, a therapeutic agent, toxin, hormone, peptide, protein, vector, siRNA, micro-RNA or nucleic acid. In some embodiments, the solid support can be beads, biosensors, nanoparticles, microchannels, microarrays, and microfluidic devices, glass slides, glass chambers, or gold particles. In some embodiments, the enzyme can be alkaline phosphatase (AP), horse-raddish-peroxidase (HRP), Luciferase, and beta-galactosidase. In some embodiments, the bead can be 1-200 micrometer in diameter, preferably 1-10 micrometer in diameter.

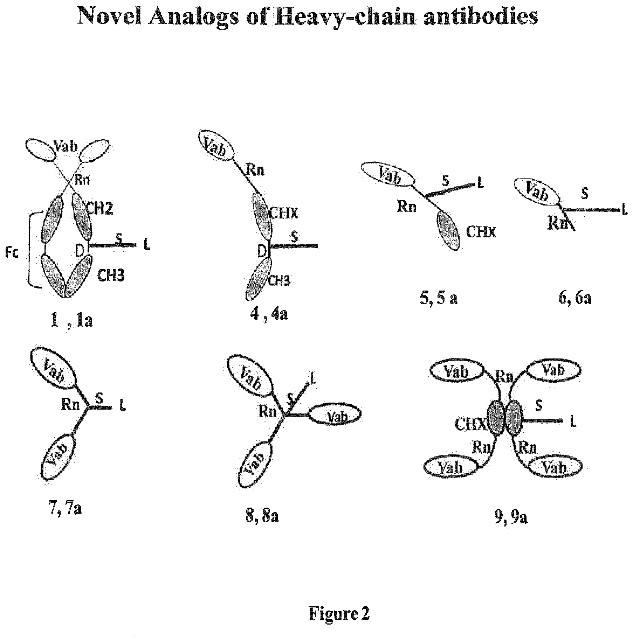

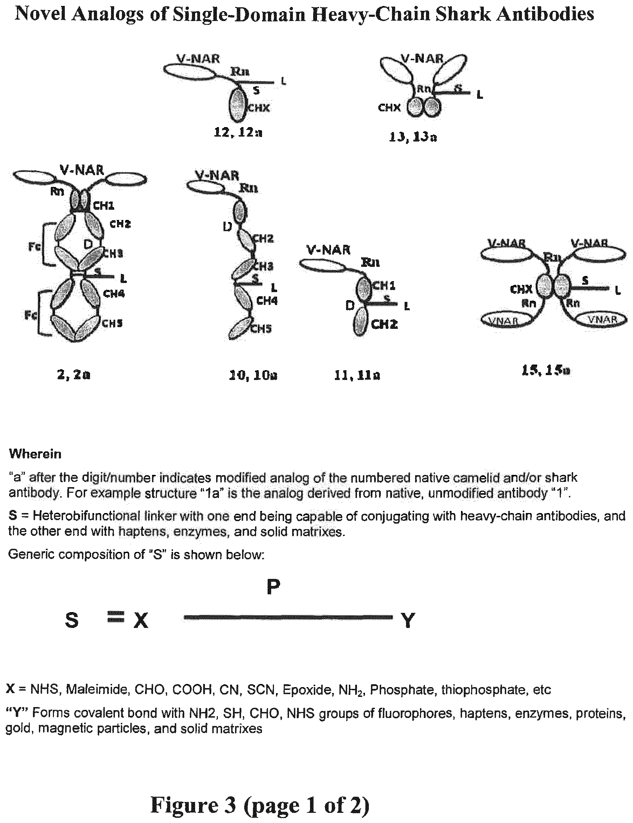

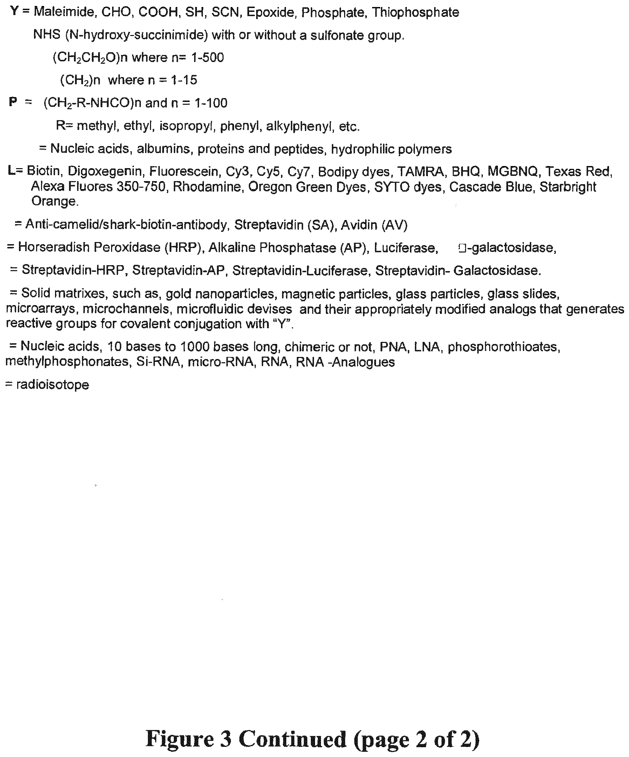

In some embodiments, the polypeptides or the compositions of the above aspects of the invention have structures 1, 1a, 4, 4a, 5, 5a, 6, 6a, 7,7a, 8, 8a, 9, 9a, 10, 10a (Figure 2), wherein "a" represents analog of the unmodified parent antibody. For example, 1, 1a represent native unmodified structure 1 without "S" and "L" whereas 1a contains modified structure 1 comprising of "S" and "L". Also, "CHX" in figures 2 and 3 represents at least ten contiguous amino acids derived from a source other than camelid and/ or shark single-domain heavy chain antibodies lacking light-chains; "S" represents a linker; "Rn" represents all or a portion of at least one camelid or shark hinge region of single domain heavy chain antibody; "L" represents an entity linked to the polypeptide, and Vab represents camelid or shark variable region of single domain heavy chain antibody, "D" represents at least two amino acids comprising at least one charged amino acid between the two domains of the camelid and shark antibodies.

In other embodiments, the polypeptides or the compositions of the above aspects of the invention have structures 2, 2a, 11, 11a, 12, 12a, 13, 13a, 14, 14a, and 15, 15a. (Figure 3), wherein "a" represents analog of the unmodified parent antibody. For example, 2, represents native unmodified structure 2 and 2a represents modified structure with "S" and "L" Also, "CHX" in figures 2 and 3 represents at least ten contiguous amino acids derived from a source other than camelid and/ or shark single-domain heavy chain antibodies lacking light-chains; "S" represents a linker; "Rn" represents all or a portion of at least one camelid or shark hinge region of single domain heavy chain antibody; L represents an entity linked to the polypeptide, and Vab represents camelid or shark variable region of single domain heavy chain antibody, "D" represents at least two amino acids comprising at least one charged amino acid, VNAR represents shark variable antigen-binding region of single domain heavy chain only shark antibody without the light-chains. CH1, CH2, CH3, CH4 and CH5 represent five constant domains of shark antibodies.

In one embodiment, the generic composition of the antibody polypeptide is represented by:

**[Vab]m- S-L**

in which
Vab = Variable antigen-binding domain of camelid and/or shark single domain heavy chain antibodies;
m = 1 to 10, preferably 2 to 5 such that the MW is approximately between 15 to 65 KDa for optimal biodistribution and retention in the body;
"S" is selected from the group consisting of groups I and II in which group I includes 1-20 amino acids of the hinge region of camelid and /or shark single domain heavy chain antibodies comprising at least one lysine and /or cysteine, and group II includes hetrobifunctional linker with one end being capable of covalent binding with amino- or aldehyde group of single-domain antibodies, and the other end with an entity "L";
"L" represents an entity linked to Vab domain. "L" can be a detectable label, enzyme or protein (for example, horse radish peroxidase, alkaline phosphatase, luciferase, beta-galactosidase, and streptavidin), antibody, nucleic acid (for example, DNA, Modified DNA, Locked-DNA, PNA (Peptide Nucleic Acids), RNA, Si-RNA, Micro-RNA( MiRNA), mRNA, RNA-Conjugates/ Modifications), radionucleotides (for example, Fluorine-18, Gallium-67, Krypton-81m, Rubidium-82, Technetium-99m, Indium-111, Iodine-123, Xenon-133, and Thallium-201, Yttrium-90, and Iodine-131), toxins (for example, Immunotoxins, Ricin, Saporin, Maytansinoid, and Calicheamicin), solid support (for example, Microchannels, Microfluidic Device, Microarrays, Biosensors, Glass Slides, Glass Chambers, Magnetic Beads, and Gold Nanoparticles), and therapeutic agents (for example, nucleolytic enzymes, antibiotics, and chemotherapeutic agents such as Paclitaxel its derivatives).

In one embodiment, the generic composition of "S" is S = X-P-Y in which X can be of NHS (N-Hydroxy-Succinimide), sulfo-NHS, CHO, COOH, CN, SCN, epoxide, phosphate and other moieties capable of forming covalent bond with NH2 groups of single-domain antibodies; Y can be maleimido, NHS, sulfo-NHS, SH, COOH, SCN, NH2, and epoxide, capable of forming a covalent bond with the thiol group of the detectable label; P can be (CH₂CH₂O)n, wherein n= 1-500; DNA, modified DNA, modified RNA; (CH₂)n¹, wherein n¹ = 1-15; (Ra-NHCO)n², wherein n² = 1-100; Ra = charged amino acid; nucleic acids; nylon, polystyrene; polypropylene; protein; and chimeric protein-nucleic acids.

In some embodiments, the disease may be cancer, Parkinson's disease, Alzheimer's disease, AIDS, Lyme disease, malaria, SARS, Down syndrome, anthrax, salmonella or bacterial botulism, staphylococcus aureus. In some embodiments, the cancer can be lung cancer, bladder cancer, gastric cancer, ovarian cancer, brain cancer, breast cancer, prostate cancer, cervical cancer, ovarian cancer, oral cancer, colorectal cancer, leukemia, childhood neuroblastoma, or Non-Hodgkin's lymphoma.

In some embodiments of the above aspects of the invention, the polypeptide can bind specifically one or more biomarkers. Exemplary biomarkers include AMACR, TMPRSS2-ERG, HAAH, APP, Aβ42, ALZAS, Tau, gamma secretase, beta secretase, PEDF, BDNF, Cystatin C, VGF nerve growth factor inducible, APO-E, GSK-3 binding protein, TEM1, PGD2, EGFR, ESR-1, HER-2/neu, P53, RAS, SMAD4, Smad7, TNF-alfa, HPV, tPA, PCA-3, Mucin, Cadherin-2, FcRn alpha chain, cytokeratins 1-20, Apo-H, Celuloplasmin, Apo AII, VGF, Vif, LEDGF/p75, TS101, gp120, CCR5, HIV protease, HIV integrase, Bacillus anthracis protein, NadD (Nicotinate Mononucletide Adenyltransferase), Plasmodium falciparum, cGMP directed phosphodiestrase, chain B of Clostridium botulinum neurotroxin type E protein, Borrelia VlsE protein, ACE2 receptor, SFRS4, or SAMP.

In some embodiments of the above aspects of the invention, the polypeptide may specifically bind to the biomarkers associated with Alzheimer's disease. Exemplary biomarkers associated with Alzheimer's disease include but are not limited to Amyloid-beta, ALZAS, Tau, Cyclophilin-D (Cyp-D), Abeta binding alcohol dehydrogenase (ABAD), N-methyl-D-aspartate receptor (NMDAR), mSOD1, mHTT (mutant huntingtin), 3-NP, phosphatidylserine (PtDS), MPTP, integrin α4β1, integrin- α4β7, PPAR-γ, MAdCAM-1, DJ-1, Bax-1, PEDF, HPX, Cystatin-C, Beta-2-Microglobulin, BDNF, Tau-Kinase, γ-Secretase, β-Secretase, Apo-E4, and VGF-Peptide, TOM, hPReP, PLSCR1, integrin- DJ-1, and enzymes involved in the mitochondrial and myelin dysfunction.

In some embodiments of the above aspects of the invention, the polypeptide may specifically bind to the biomarkers associated with Parkinson's disease. Exemplary biomarkers associated with Parkinson's disease include but are not limited to Apo-H, Cerulopasmin, Chromogranin-B, VDBP, Apo-E, Apo-AII, and alfa-Synuclein.

In some embodiments of the above aspects of the invention, the polypeptide may specifically bind to the biomarkers associated with brain cancer. Exemplary biomarkers associated with brain cancer include but are not limited to TEM1, Plasmalemmal Vesicle (PV-1), Prostaglandin D Synthetase, and (PGD-S).

In some embodiments of the above aspects of the invention, the polypeptide may specifically bind to the biomarkers associated with HIV/AIDS. Exemplary biomarkers associated with HIV/AIDS include but are not limited to gp120, Vif, LEDGF/p75, TS101, HIV-Integrase, HIV-Reverse Transcriptase, HIV-Protease, CCR5, and CXCR4.

In some embodiments of the above aspects of the invention, the polypeptide may specifically bind to the biomarkers associated with lung cancer. Exemplary biomarkers associated with lung cancer include but are not limited to KRAS, Ki67, EGFR, KLKB1, EpCAM, CYFRA21-1, tPA, ProGRP, Neuron-specific Enolase (NSE), and hnRNP.

In some embodiments of the above aspects of the invention, the polypeptide may specifically bind to the biomarkers associated with prostate cancer. Exemplary biomarkers associated with prostate cancer include but are not limited to AMACR, PCA3, TMPRSS2-ERG, HEPSIN, B7-H3, SSeCKs, EPCA-2, PSMA, BAG-1, PSA, MUC6, hK2, PCA1, PCNA, RKIP, and c-HGK.

In some embodiments of the above aspects of the invention, the polypeptide may specifically bind to the biomarkers associated with breast cancer. Exemplary biomarkers associated with breast cancer include but are not limited to EGFR, EGFRT790M, HER-2, Notch-4, ALDH-1, ESR1, SBEM, HSP70, hK-10, MSA, p53, MMP-2, PTEN, Pepsinigen-C, Sigma-S, Topo-11-alfauKPA, BRCA-1, BRCA-2, SCGB2A1, and SCGB1D2.

In some embodiments of the above aspects of the invention, the polypeptide may specifically bind to the biomarkers associated with colorectal cancer. Exemplary biomarkers associated with colorectal cancer include but are not limited to SMAD4, EGFR, KRAS, p53, TS, MSI-H, REGIA, EXTL3, plK3CA, VEGF, HAAH, EpCAM, TEM8, TK1, STAT-3, SMAD-7, beta-Catenin, CK20, MMP-1, MMP-2, MMP-7,9,11, and VEGF-D.

In some embodiments of the above aspects of the invention, the polypeptide may specifically bind to the biomarkers associated with ovarian cancer. Exemplary biomarkers associated with ovarian cancer include but are not limited to CD24, CD34, EpCAM, hK8, 10, 13, CKB, Cathesin B, M-CAM, c-ETS1, and EMMPRIN.

In some embodiments of the above aspects of the invention, the polypeptide may specifically bind to the biomarkers associated with cervical cancer. Exemplary biomarkers associated with cervical cancer include but are not limited to HPV, CD34, ERCC1, Beta-CF, Id-1, UGF, SCC, p16, p21WAF1, PP-4, and TPS.

In some embodiments of the above aspects of the invention, the polypeptide may specifically bind to the biomarkers associated with bladder cancer. Exemplary biomarkers associated with bladder cancer include but are not limited to CK18, CK20, BLCal, BLCA-4, CYFRA21-1, TFT, BTA, Survivin, UCA1, UPII, FAS, and DD23.

In some embodiments of the above aspects of the invention, the polypeptide may specifically bind to the biomarkers associated with a pathogenic bacteria. Exemplary pathogenic bacteria include but are not limited to Clostridium Botulinum (Bacterial Botulism), Bacillus Anthracis (Anthrax), Salmonella Typhi (Typhoid Fever), Treponema Pallidum (Syphilis), Plasmodinum (Malaria), Chlamadyia (STDs), Borrelia B (Lyme disease), Staphyloccus Aureus, Tetanus, Meningococcal Meningitis (Bacterial Meningitis), and Mycobacterium tuberculosis (Tuberculosis, TB), and NadD (Nicotinate Mononucleotide Adenyltransferase, an enzyme involved in inducing resistance to antibiotics).

In some embodiments of the above aspects of the invention, the polypeptide may specifically bind to the biomarkers associated with a pathogenic virus. Exemplary pathogenic virus include but are not limited to Pandemic Flu Virus H1N1 strain, Influenza virus H5N1 strain, Hepatitis B virus (HBV) antigen OSt-577, HBV core antigen HBcAg (HBV), HBV antigen Wnt-1, Hepatitis C Virus (HCV) antigen Wnt-1, and HCV RNA (HCV).

In some embodiments, the antibody is produced using chemical methods as described in pending US Patent Application # 12563330. Briefly, the method includes a chemical synthesis of a polypeptide comprising one, two, or more variable antigen-binding (Vab) domains using the parent antibody produced from camelid and /or shark as a starting material for generating the polypeptide with one or more Vab domains.

Still in another embodiment, the invention provides a method for generating polypeptides comprising multivalent variable antigen-binding domains improving binding affinity between antibody and its antigen.

In some embodiments, the antibody is produced using recombinant DNA methods as described in pending US Patent Application # 12563330. Briefly, the method includes isolating the RNA from lymphocytes, reverse-transcription with oligo-dT priming, amplification of the generated cDNA encoding the camelid or shark antibody, inserting the amplified DNA in a phage-display vector, transforming the E.Coli cells, and selecting the clones that express highly specific antibodies.

The term "antibody" as used herein refers to immunoglobulin G (IgG) having only heavy chains without the light-chain and also constant domain 1 (CH1) in case of camelid antibodies. The shark antibody without the light-chains is known in the art as shark IgNAR. An antibody of this invention can be monoclonal or polyclonal.

The term "analog" within the scope of the term "antibody" include those produced by digestion with various proteases, those produced by chemical cleavage, chemical coupling, chemical conjugation, and those produced recombinantly, so long as the fragment remains capable of specific binding to a target molecule. Analogs within the scope of the term include antibodies (or fragments thereof) that have been modified in sequence, but remain capable of specific binding to a target molecule, including: interspecies chimeric and humanized antibodies; antibody fusions; heteromeric antibody complexes and antibody fusions, such as diabodies (bispecific antibodies), single-chain diabodies, and intrabodies (see, e.g., Marasco (ed.), Intracellular Antibodies: Research and Disease Applications, Springer-Verlag New York, Inc. (1998) (ISBN: 3540641513). As used herein, antibodies can be produced by any known technique, including harvest from cell culture of native B lymphocytes, harvest from culture of hybridomas, recombinant expression systems, and phage display.

The terms "heavy chain only antibody" and "single domain heavy chain antibody" has been used herein interchangeably in the context of camelid and shark antibodies and refer to camelid immunoglobulin G (IgG) and shark IgNAR having only heavy chains without the heavy chain constant domain 1 (CH1) and further lacking the light chain such as camelids IgG2 and IgG3 and shark IgNAR. Heavy chain only antibody can be monoclonal or polyclonal.

The term "improved biodistribution and retention" as used herein in the context of polypeptides, antibodies and its analogs refers to polypeptides, antibodies and its analogs that can cross cell membrane and blood brain barrier (BBB) and have greater thermal and chemical stability than conventional immunoglobulin G with heavy and light chains. Typically such polypeptides, antibodies and its analogs have molecular weight between 25 to 90 KDa, preferably between 30 to 60 KDa. In some embodiments, the molecular weight is at least 25 KDa, 30 KDa, 35 KDa, 40 KDa, 45 KDa, 50 KDa, 55 KDa, 60 KDa, 65 KDa, 70 KDa, 75 KDa, 80 KDa, 85 KDa, or 90 KDa. Although larger and smaller molecular weights are possible.

The term "specifically binds to" as used herein in the context of an antibody or its analogs refers to binding of an antibody or its analogs specifically to an epitope such that the antibody or its analog can distinguish between two proteins with and without such epitope.

The terms "biomarker" and antigen is used interchangeably and refer to a molecule or group of molecules comprised of nucleic acids, carbohydrates, lipids, proteins, peptides, enzymes and antibodies which is associated with a disease, physiological condition, or an organism. An organism can be pathogenic or nonpathogenic. A biomarker may not necessarily be the reason for a disease or a physiological condition. An amount of a biomarker may be increased or decreased in disease or a physiological condition.

The term "camelid" as used herein refers to members of the biological family Camelidae in the Order: Artiodactyla, Suborder: Tylopoda. Exemplary members of this group include camels, dromedaries, llamas, alpacas, vicuñas, and guanacos.

The term "shark" as used herein refers to members that belong to the super order Selachimorpha in the subclass Elasmobranchii in the class Chondrichthyes. There are more than 400 species of sharks known. Exemplary members of the class Chondrichthyes include great white sharks, houndsharks, cat sharks, hammerhead sharks, blue, tiger, bull, grey reef, blacktip reef, Caribbean reef, blacktail reef, whitetip reef, oceanic whitetip sharks, zebra sharks, nurse sharks, wobbegongs, bramble sharks, dogfish, roughsharks, and prickly sharks.

The term "a portion of" in the context of antibodies such as camelid and shark heavy chain only antibodies and their analogs, or human antibodies means at least 2, 5, 10, 15, 20, 25, 30, 35, 40, 45, 50, 75, 100, 125, 150, 200, 250, 300, 350, 400 or more amino acids.

The term "a portion of" in the context of hinge region of camelid and shark single domain heavy chain antibodies means at least 1, 2, 5, 15, 20, 25, 30, 35, 40, 45, 50, 75, 100, 125, 150, 200, or more amino acids of the hinge region.

The terms "diagnose" or "diagnosis" as used herein refers to the act or process of identifying or determining a disease or condition in an organism or the cause of a disease or condition by the evaluation of the signs and symptoms of the disease or disorder. Usually, a diagnosis of a disease or disorder is based on the evaluation of one or more factors and/or symptoms that are indicative of the disease. That is, a diagnosis can be made based on the presence, absence or amount of a factor which is indicative of presence or absence of the disease or condition. Each factor or symptom that is considered to be indicative for the diagnosis of a particular disease does not need be exclusively related to the particular disease; i.e. there may be differential diagnoses that can be inferred from a diagnostic factor or symptom. Likewise, there may be instances where a factor or symptom that is indicative of a particular disease is present in an individual that does not have the particular disease.

The term "reference value" as used herein means a value which can be used for comparison with a biomarker under investigation. In one case, a reference value may be the level of a biomarker under investigation from one or more individuals without any known disease. In another case, a reference value may be the level of the biomarker in an individual's sample collected at a different time.

"Sample" or "patient sample" as used herein includes biological samples such as cells, tissues, bodily fluids, and stool. "Bodily fluids" may include, but are not limited to, blood, serum, plasma, saliva, cerebral spinal fluid, pleural fluid, tears, lactal duct fluid, lymph, sputum, urine, amniotic fluid, and semen. A sample may include a bodily fluid that is "acellular". An "acellular bodily fluid" includes less than about 1% (w/w) whole cellular material. Plasma or serum are examples of acellular bodily fluids. A sample may include a specimen of natural or synthetic origin.

The term "body fluid" or "bodily fluid" as used herein refers to any fluid from the body of an animal. Examples of body fluids include, but are not limited to, plasma, serum, blood, lymphatic fluid, cerebrospinal fluid, synovial fluid, urine, saliva, mucous, phlegm and sputum. A body fluid sample may be collected by any suitable method. The body fluid sample may be used immediately or may be stored for later use. Any suitable storage method known in the art may be used to store the body fluid sample; for example, the sample may be frozen at about 20°C to about -70°C. Suitable body fluids are acellular fluids. "Acellular" fluids include body fluid samples in which cells are absent or are present in such low amounts that the peptidase activity level determined reflects its level in the liquid portion of the sample, rather than in the cellular portion. Typically, an acellular body fluid contains no intact cells. Examples of acellular fluids include plasma or serum, or body fluids from which cells have been removed.

The term "enzyme linked immunosorbent assay" (ELISA) as used herein refers to an antibody-based assay in which detection of the antigen of interest is accomplished via an enzymatic reaction producing a detectable signal. ELISA can be run as a competitive or noncompetitive format. ELISA also includes a 2-site or "sandwich" assay in which two antibodies to the antigen are used, one antibody to capture the antigen and one labeled with an enzyme or other detectable label to detect captured antibody-antigen complex. In a typical 2-site ELISA, the antigen has at least one epitope to which unlabeled antibody and an enzyme-linked antibody can bind with high affinity. An antigen can thus be affinity captured and detected using an enzyme-linked antibody. Typical enzymes of choice include alkaline phosphatase or horseradish peroxidase, both of which generated a detectable product upon digestion of appropriate substrates.

The term "label" as used herein, refers to any physical molecule directly or indirectly associated with a specific binding agent or antigen which provides a means for detection for that antibody or antigen. A "detectable label" as used herein refers any moiety used to achieve signal to measure the amount of complex formation between a target and a binding agent. These labels are detectable by spectroscopic, photochemical, biochemical, immunochemical, electromagnetic, radiochemical, or chemical means, such as fluorescence, chemifluoresence, or chemiluminescence, electro-chemiluminescence or any other appropriate means. Suitable detectable labels include fluorescent dye molecules or fluorophores.

The terms "polypeptide," "protein," and "peptide" are used herein interchangeably to refer to amino acid chains in which the amino acid residues are linked by peptide bonds or modified peptide bonds. The amino acid chains can be of any length of greater than two amino acids. Unless otherwise specified, the terms "polypeptide," "protein," and "peptide" also encompass various modified forms thereof. Such modified forms may be naturally occurring modified forms or chemically modified forms. Examples of modified forms include, but are not limited to, glycosylated forms, phosphorylated forms, myristoylated forms, palmitoylated forms, ribosylated forms, acetylated forms, ubiquitinated forms, etc. Modifications also include intramolecular crosslinking and covalent attachment to various moieties such as lipids, flavin, biotin, polyethylene glycol or derivatives thereof, etc. In addition, modifications may also include cyclization, branching and cross-linking. Further, amino acids other than the conventional twenty amino acids encoded by genes may also be included in a polypeptide.

The term "detectable label" as used herein in the context of antibody or its analogs refers to a molecule or a compound or a group of molecules or a group of compounds associated with a binding agent such as an antibody or its analogs, secondary antibody and is used to identify the binding agent bound to its target such as an antigen, primary antibody. A detectable label can also be used in to detect nucleic acids. In such cases a detectable label may be incorporated into a nucleic acid during amplification reactions or a detectable label may be associated a probe to detect the nucleic acid.

The terms "ultrasensitive" or "ultrasensitivity" as used herein in the context of antibodies refers to the detection of fewer than 200 molecules of the pathogenic proteins from patient's sample.

"Detecting" as used herein in context of detecting a signal from a detectable label to indicate the presence of a nucleic acid of interest in the sample (or the presence or absence of a protein of interest in the sample) does not require the method to provide 100% sensitivity and/or 100% specificity. As is well known, "sensitivity" is the probability that a test is positive, given that the person has a genomic nucleic acid sequence, while "specificity" is the probability that a test is negative, given that the person does not have the genomic nucleic acid sequence. A sensitivity of at least 50% is preferred, although sensitivities of at least 60%, at least 70%, at least 80%, at least 90% and at least 99% are clearly more preferred. A specificity of at least 50% is preferred, although specificity of at least 60%, at least 70%, at least 80%, at least 90% and at least 99% are clearly more preferred. Detecting also encompasses assays with false positives and false negatives. False negative rates may be 1%, 5%, 10%, 15%, 20% or even higher. False positive rates may be 1%, 5%, 10%, 15%, 20% or even higher.

The term "about" as used herein in reference to quantitative measurements or values, refers to the indicated value plus or minus 10%.

"Nucleic acid" as used herein refers to an oligonucleotide, nucleotide or polynucleotide, and fragments or portions thereof, which may be single or double stranded, and represent the sense or antisense strand. A nucleic acid may include DNA or RNA, and may be of natural or synthetic origin and may contain deoxyribonuclcotides, ribonucleotides, or nucleotide analogs in any combination.

Non-limiting examples of polynucleotides include a gene or gene fragment, genomic DNA, exons, introns, mRNA, tRNA, rRNA, ribozymes, cDNA, recombinant polynucleotides, branched polynucleotides, plasmids, vectors, isolated DNA of any sequence, isolated RNA of any sequence, synthetic nucleic acid, nucleic acid probes and primers. Polynucleotides may be natural or synthetic. Polynucleotide may comprise modified nucleotides, such as methylated nucleotides and nucleotide analogs, uracyl, other sugars and linking groups such as fluororibose and thiolate, and nucleotide branches. A nucleic acid may be modified such as by conjugation, with a labeling component. Other types of modifications included in this definition are caps, substitution of one or more of the naturally occurring nucleotides with an analog, and introduction of chemical entities for attaching the polynucleotide to other molecules such as proteins, metal ions, labeling components, other polynucleotides or a solid support. Nucleic acid may include nucleic acid that has been amplified (e.g., using polymerase chain reaction).

A fragment of a nucleic acid generally contains at least about 15, 20, 25, 30, 35, 40, 45, 50, 75, 100, 200, 300, 400, 500, 1000 nucleotides or more. Larger fragments are possible and may include about 2,000, 2,500, 3,000, 3,500, 4,000, 5,000 7,500, or 10,000 bases.

"Gene" as used herein refers to a DNA sequence that comprises control and coding sequences necessary for the production of an RNA, which may have a noncoding function (e.g., a ribosomal or transfer RNA) or which may include a polypeptide or a polypeptide precursor. The RNA or polypeptide may be encoded by a full length coding sequence or by any portion of the coding sequence so long as the desired activity or function is retained.

"cDNA" as used herein refers to complementary or copy polynucleotide produced from an RNA template by the action of RNA-dependent DNA polymerase activity (e.g., reverse transcriptase). cDNA can be single stranded, double stranded or partially double stranded.

cDNA may contain unnatural nucleotides. cDNA can be modified after being synthesized. cDNA may comprise a detectable label.

As used herein, "subject" or "individual" is meant a human or any other animal that has cells. A subject can be a patient, which refers to a human presenting to a medical provider for diagnosis or treatment of a disease. A human includes pre and post natal forms.

The term "patient" as used herein, refers to one who receives medical care, attention or treatment. As used herein, the term is meant to encompass a person diagnosed with a disease as well as a person who may be symptomatic for a disease but who has not yet been diagnosed.

The term "vector or phagemid" as used herein refers to a recombinant DNA or RNA plasmid or virus that comprises a heterologous polynucleotide capable of being delivered to a target cell, either in vitro, in vivo or ex-vivo. The heterologous polynucleotide can comprise a sequence of interest and can be operably linked to another nucleic acid sequence such as promoter or enhancer and may control the transcription of the nucleic acid sequence of interest. As used herein, a vector need not be capable of replication in the ultimate target cell or subject. The term vector may include expression vector and cloning vector.

Suitable expression vectors are well-known in the art, and include vectors capable of expressing a polynucleotide operatively linked to a regulatory sequence, such as a promoter region that is capable of regulating expression of such DNA. Thus, an expression vector refers to a recombinant DNA or RNA construct, such as a plasmid, a phage, recombinant virus or other vector that, upon introduction into an appropriate host cell, results in expression of the inserted DNA. Appropriate expression vectors include those that are replicable in eukaryotic cells and/or prokaryotic cells and those that remain episomal or those which integrate into the host cell genome.

The term "promoter" as used herein refers to a segment of DNA that controls transcription of polynucleotide to which it is operatively linked. Promoters, depending upon the nature of the regulation, may be constitutive or regulated. Exemplary eukaryotic promoters contemplated for use in the practice of the present invention include the SV40 early promoter, the cytomegalovirus (CMV) promoter, the mouse mammary tumor virus (MMTV) steroid-inducible promoter, Moloney murine leukemia virus (MMLV) promoter. Exemplary promoters suitable for use with prokaryotic hosts include T7 promoter, beta-lactamase promoter, lactose promoter systems, alkaline phosphatase promoter, a tryptophan (trp) promoter system, and hybrid promoters such as the lac promoter.

The term "antibody" as used herein refers to immunoglobulin G (IgG) having only heavy chains without the heavy chain constant domain 1 (CH1) and also lacking the light chain such as in shark IgNAR and camelids IgG2 and IgG3. Antibody can be monoclonal or polyclonal.

The term "analog" within the scope of the term "antibody" include those produced by digestion with various proteases, those produced by chemical cleavage, chemical coupling, chemical conjugation, and those produced recombinantly, so long as the fragment remains capable of specific binding to a target molecule. Analogs within the scope of the term include antibodies (or fragments thereof) that have been modified in sequence, but remain capable of specific binding to a target molecule, including: interspecies chimeric and humanized antibodies; antibody fusions; heteromeric antibody complexes and antibody fusions, such as diabodies (bispecific antibodies), single-chain diabodies, and intrabodies (see, e.g., Marasco (ed.), Intracellular Antibodies: Research and Disease Applications, Springer-Verlag New York, Inc. (1998) (ISBN: 3540641513). As used herein, antibodies can be produced by any known technique, including harvest from cell culture of native B lymphocytes, harvest from culture of hybridomas, recombinant expression systems, and phage display.

The terms "heavy chain only antibody" and "single domain heavy chain antibody" has been used herein interchangeably in the context of camelid and shark antibodies and refer to camelid immunoglobulin G (IgG) and shark IgNAR having only heavy chains without the heavy chain constant domain 1 (CH1) and further lacking the light chain such as camelids IgG2 and IgG3 and shark IgNAR. Heavy chain only antibody can be monoclonal or polyclonal.

Unless otherwise specified, the terms "a" or "an" mean "one or more" throughout this application.

### BRIEF DESCRIPTION OF THE FIGURES

FIG. 1 shows structural differences between camel, shark, and mouse immunoglobulins (IgGs). The notations CH1, CH2, CH3, CH4, CH5 represent constant domain 2, 3, 4 of single domain heavy chain antibody of the respective species. The notations Vab and VNAR represent variable domain of camelid and shark single domain heavy chain antibodies respectively.
FIG. 2 shows the structure of exemplary analogs of camelid single-domain antibodies without the light-chains: mini-antibody 1 and its analogs 1a; micro-antibody 4 and its analogs 4a; sub-nano-antibody 5 and its analogs 5a; nano-antibody 6 and its analogs 6a; dimeric nano-antibody 7 and its analogs 7a; trimeri-nanoantibody 8 and its analogs; and tetrameric nano-antibody 9 and its analogs 9a. The notation "Rn" represents all or portion of the hinge region of camelid or shark single domain antibodies. CHX represents segment of human IgG CH1 domain or CH2 domain of camelid antibody. "S" stands for a spacer or linker. "L" is a ligand.
FIG. 3 shows the structure of exemplary analogs of shark single-domain antibodies without the light-chains: Hark IgNAR 2 and its analogs 2a; shark mini-antibody 11 and its analogs 11a; shark micro-antibody 12 and its analogs 12a; shark sub-nano-antibody 13 and its analogs 13a; shark dimeric nano-antibody 14 and its analogs 14a; and shark tetrameric nano-antibody 15 and 15a. The notation "Rn" represents all or portion of the hinge region of shark single domain antibodies. CHX represents segment of human IgG or CH1 domain of shark antibody. "S" stands for a spacer or linker. "L" is a ligand.
Figure 4 shows the steps involved in the chemical synthesis of exemplary analogs represented by structures 1a and 4a, respectively, of camelid mini-antibody 1 and micro-antibody 4. The notation "Rn" represents all or portion of the hinge region of camelid or shark single domain antibodies.
Figure 5 shows the steps involved in the chemical transformation of exemplary sub-nano-antibody 5 into its analogs represented by structure 5a, and the synthesis of dimeric camelid nano-antibody 7 and its analogs represented by generic structure 7a.
Figure 6 shows the steps involved in the transformation of exemplary camelid dimeric nano-antibody 7 into trimeric and tetrameric nano-antibodies. The notation "Rn" represents all or portion of the hinge region of camelid or shark single domain antibodies.
Figure 7_shows the steps involved in the cloning and expression of exemplary shark IgNAR 2, exemplary shark micro-antibody 12, exemplary shark sub-nano-antibody 13 and shark-nano-antibody 30. The notation "Rn" represents all or portion of the hinge region of camelid or shark single domain antibodies.
Figure 8 shows the steps involved in the chemical synthesis of exemplary analogs of shark antibodies without the light chains: Shark IgNAR analogs represented by structure 2a; shark mini-antibody analogs represented by structure 11a; shark micro-antibody analogs represented by structure 12a; shark sub-nano-antibody analogs represented by 13a; shark dimeric nano-antibody analogs represented by 14a; and shark tetrameric -nano-antibody analogs represented by 15a. The notation "Rn" represents all or portion of the hinge region of camelid or shark single domain antibodies.
Figure 9 shows the steps involved in the chemical synthesis of exemplary shark dimeric nano-antibody 14 and its conversion into exemplary shark trimeric and tetrameric nano-antibodies 32 and 31.
Figure 10 shows the steps involved in the immobilization of exemplary single-domain camelid and shark antibodies deprived of light chains having the structures 1, 2, 4, 5, 6, 7, 8, 9, 11, 12, 13, 14, 15, 19, 20, 31, and 32.
FIG. 11 shows an exemplary scheme of capturing and detecting antigens / biomarkers associated with a disease using camelid and shark heavy chain only antibodies and their analogs.
FIG. 12 shows an exemplary scheme of capturing and detecting antigens/biomarkers associated with a disease using immobilized shark single-domain IgNAR and their analogs.
FIG. 13 shows an exemplary scheme of capturing and detecting < 200 copies of antigens/biomarkers associated with a disease using camelid and shark heavy chain only antibodies and their analogs using immuno-PCR.
Figure 14 shows an exemplary scheme of capturing and detecting circulating tumor cells from bodily fluid using camelid and shark antibodies.
FIG. 15 shows an exemplary scheme of detecting prenatal genetic disorder using captured circulating fetal cells using camelid and shark heavy chain only antibodies and their analogs.
FIG. 16 shows an exemplary scheme of detecting chromosomal translocation using captured circulating tumor cells using camelid and shark heavy chain only antibodies and their analogs.
FIG. 17 shows an exemplary nucleic acid sequence encoding human Cyclophilin D.
FIG. 18 shows an exemplary nucleic acid sequence encoding alpha beta binding Mitochondrial Alcohol Dehydrogenase (ABAD).
FIG. 19 shows an exemplary nucleic acid sequence encoding Translocase of the Outer Membrane (TOM).
FIG. 20 shows an exemplary nucleic acid sequence encoding Prosequence Protease (hPreP).
FIG. 21 shows an exemplary nucleic acid sequence encoding Homo sapiens integrin beta 1.
FIG. 22 shows an exemplary nucleic acid sequence encoding Homo sapiens mucosal vascular addressin cell adhesion molecule 1 (MADCAM1).
FIG. 23 shows an exemplary nucleic acid sequence encoding Cu/Zn-superoxide dismutase (mSOD1).
FIG. 24 shows an exemplary nucleic acid sequence encoding Mus musculus mRNA for MPTPdelta.
FIG. 25 shows an exemplary nucleic acid sequence encoding Homo sapiens huntingtin (HTT).
FIG. 26 shows an exemplary nucleic acid sequence encoding N-Methyl-D-Aspartate Receptor (NMDAR).
FIG. 27 shows an exemplary nucleic acid sequence encoding Phosphatidylserine Synthase (PTDS).

### DETAILED DESCRIPTION OF THE INVENTION

The present invention discloses the use of camelid and/or shark single-domain heavy-chain only antibodies and their analogs for ultrasensitive detection of antigens. The method is useful for diagnosing human diseases at an early stage of their manifestation, when the concentration of antigens associated with such diseases is very low for example, 200 or fewer molecules in 0.1 ml of the bodily fluid. The invention also teaches methods for the development of nano-biomedical technology platforms utilizing camelid and/or shark heavy-chain only antibodies and their analogs for in-vitro diagnosis of human and animal diseases with such antibodies.

### Camelid and Shark Antibodies

The hetero-tetrameric structure of antibodies exists in humans and most animals but the single-domain heavy-chain only dimeric structure, without the light-chains, is considered characteristic of camelids and sharks [ Nature Biotechnology, 23, 1126 (2005)]. These antibodies are relatively simple molecules but with unique characteristics. Their size is about 2/3rd the size of traditional antibodies, hence a lower molecular weight (about 90 KDa), with similar antigen binding affinity, but with water solubility 100 to 1000 folds higher than the conventional antibodies. Because of the lower molecular weight, the authors of this application call these antibodies as "Single-domain Mini-antibodies" (sdMnAbs) or simply "Mini-Antibodies" (MnAbs).

Another characteristic of the single-domain antibodies derived from sharks and camelids is that they have very high thermal stability compared to the conventional mAbs. For example, camel antibodies can maintain their antigen binding ability even at 90°C [Biochim. Biophys. Acta., 141, 7 (1999)]. Furthermore, complementary determining region 3 (CDR3) of camel Vab region is longer, comprising of 16-21 amino acids, than the CDR3 of mouse VH region comprising only of 9 amino acids [Protein Engineering, 7, 1129 (1994)]. The larger length of CDR3 of camel Vab region is responsible for higher diversity of antibody repertoire of camel antibodies.

In addition to being devoid of light chains, the camel heavy-chain antibodies also lack the first domain of the constant region called CH1, though the shark antibodies do have CH1 domain and two additional constant domains CH4 and CH5 [ Nature Biotech. 23, 1126 (2005)]. Furthermore, the hinge regions of camel and shark antibodies have an amino acid sequence different from that of normal heterotetrameric conventional antibodies [(S. Muyldermans, Reviews in Mol. Biotech., 74, 277 (2001)]. Without the light chain, these antibodies bind to their antigens by the variable antigen-binding domain of the heavy-chain immunoglobulin, which is referred to as Vab by the authors of this application (VHH in the literature), to distinguish it from the variable domain VH of the conventional antibodies. The single -domain Vab is amazingly stable by itself without having to be attached to the parent antibody. This smallest intact and independently functional antigen-binding fragment Vab, with a molecular weight of ~12-15 KDa, is referred to as nano-antibody by the authors of this application. In the literature, it is known as nanobody [(S. Muyldermans, Reviews in Mol. Biotech., 74, 277 (2001)].

The genes encoding these full length single-domain heavy-chain antibodies and antibody-antigen binding fragment Vab (camel and shark) can be cloned in phage display vectors, and selection of antigen binders by panning and expression of selected VHH in bacteria offer a very good alternative procedure to produce these antibodies on a large scale. Also, only one domain has to be cloned and expressed to produce in vivo an intact, matured antigen-binding fragment.

There are structural differences between the variable regions of single domain antibodies and conventional antibodies. Conventional antibodies have three constant domains while camel has two and shark has five constant domains. The largest structural difference is, however, found between a VH (conventional antibodies) and Vab (heavy-chain only antibodies of camel and shark) (see below) at the hypervariable regions. Camelid Vab and shark V-NAR domains each display surface loops which are larger than for conventional murine and human IgGs, and are able to penetrate cavities in target antigens, such as enzyme active sites and canyons in viral and infectious disease biomarkers [PNAS USA., 101, 12444 (2004); Proteins, 55, 187 (2005)]. In human and mouse, the VH loops are folded in a limited number of canonical structures. In contrast, the antigen binding loop of Vab possess many deviations of these canonical structures that specifically bind into such active sites, therefore, represent powerful tool to modulate biological activities [( K. Decanniere et al., Structure, 7, 361 (2000)]. The high incidence of amino acid insertions or deletions, in or adjacent to first and second antigen-binding loops of Vab will undoubtedly diversify, even further, the possible antigen-binding loop conformations.

Though there are structural differences between camel and shark parent heavy-chain antibodies (FIG. 1), the antigen-antibody binding domains, Vab and V-NAR, respectively, have similar binding characteristics. The chemical and/or protease digestion of camel and shark antibodies results in Vab and V-NAR domains, with similar binding affinities to the target antigens [Nature Biotechnology, 23, 1126 (2005)].

Other structural differences are due to the hydrophilic amino acid residues which are scattered throughout the primary structure of Vab domain. These amino acid substitutions are, for example, Leu45 to R (arginine) or Leu45 to C (cysteine); Val37 to Y (Tyr); G44 to E( Glu), and W47(Trp) to G (Gly). Therefore, the solubility of Vab is much higher than the Fab fragment of conventional mouse and human antibodies.

Another characteristic feature of the structure of camelid Vab and shark V-NAR is that it often contains a cysteine residue in the CDR3 in addition to cysteines that normally exist at positions 22 and 92 of the variable region. The cysteine residues in CDR3 form S-S bonds with other cysteines in the vicinity of CDR1 or CDR2 [Protein Engineering, 7, 1129 (1994)]. CDR1 and CDR2 are determined by the germline V gene. They play important roles together with CDR3 in antigenic binding [Nature Structural Biol., 9, 803 (1996); J. Mol. Biol., 311, 123 (2001)]. Like camel CDR3, shark also has elongated CDR3 regions comprising of 16 -27 amino acids residues [Eur. J. Immunol., 35, 936 (2005)].

The germlines of dromedaries and llamas are classified according to the length of CDR2 and cysteine positions in the V region [Nguyen et al., EMBO J., 19, 921 (2000); Harmsen et al., Mol. Immun., 37, 579 (2000)].

Immunization of camels with enzymes generates heavy-chain antibodies (HCAb) significant proportions of which are known to act as competitive enzyme inhibitors that interact with the cavity of the active site [(M. Lauwereys et al., EMBO, J. 17, 3512 (1998)]. In contrast, the conventional antibodies that are competitive enzyme inhibitors cannot bind into large cavities on the antigen surface. Camel antibodies, therefore, recognize unique epitopes that are out of reach for conventional antibodies.

Production of inhibitory recombinant Vab that bind specifically into cavities on the surface of variety of enzymes, namely, lysozyme, carbonic anhydrase, alfa-amylase, and beta- lactamase has been achieved [M. Lauwereys, et al., EMBO, J. 17, 3512 (1998)]. Hepatitis C protease inhibitor from the camelised human VH has been isolated against an 11 amino acid sequence of the viral protease [F. Martin et al., Prot. Eng., 10, 607 (1997)].

### Novel Analogs of Single-Domain Heavy-Chain Camelid and Shark Antibodies:

Figures 2 and 3 outlines the analogs of new generation of camelid and shark antibodies and their analogs, respectively, which will assist us to develop ultrasensitive and ultraspecific diagnostic assays for the detection /identification of the pathological proteins and antigens.

### Production of Parent Single-Domain Heavy-Chain Mini-Antibodies (sdmnAbs) of Structure 1

Host animals such as camel, llama, or alpaca will be immunized with the desired antigen(s), for example HER-2 protein, a biomarker for breast cancer or Aβ42 antigenic peptide for detecting amyloid plaque, following the procedures described by Murphy et al, in 1989 [Am. J. Vet. Res., 50, 1279 (1989)], but with slight modification. Immunization of camels will be done with 250 ug antigenic peptide per injection will be used, followed by 4 booster shots every two weeks 4 weeks after the initial injection. For baby sharks, 10 ug antigen/injection will be used. One antigen per animal for immunization will be used, though it may be feasible to immunize an animal simultaneously with multiple antigens to raise an immune response to each antigen separately, which can make the production cost effective [EMBO, J., 17, 3512 (1998); J. Immunol. Methods, 240,185 (2000)].

After immunization, 100 ml camel blood (or 5 ml from shark) will be withdrawn from the animal and the total IgGs will be precipitated out using ammonium sulfate precipitation procedure. Using size exclusion chromatography over Sephadex G-25, the conventional IgGs, MW 150 KDa, will be removed from the single-domain mini-IgG, 1, with MW of 90 to 100 K Da. Affinity purification to obtain high affinity sdmnAb, I, will be done by magnetic beads coated with the antigenic peptide.

### Recombinant Production of Camelid and Shark Single-Domain Antibodies:

Recombinant production of single-domain heavy-chain parent camelid antibodies 1, 4, 5, 6, 7, 8 ,9 (figure 2) and shark antibodies 2, 11, 12, 13, 14, and 15 will be done according to protocols and procedures described in pending US Patent Application 12/563,330.

### Chemical Synthesis of Analogs of Single-Domain Camel Antibodies Derivatization and Immobilization of Camelid Mini-antibody 1:

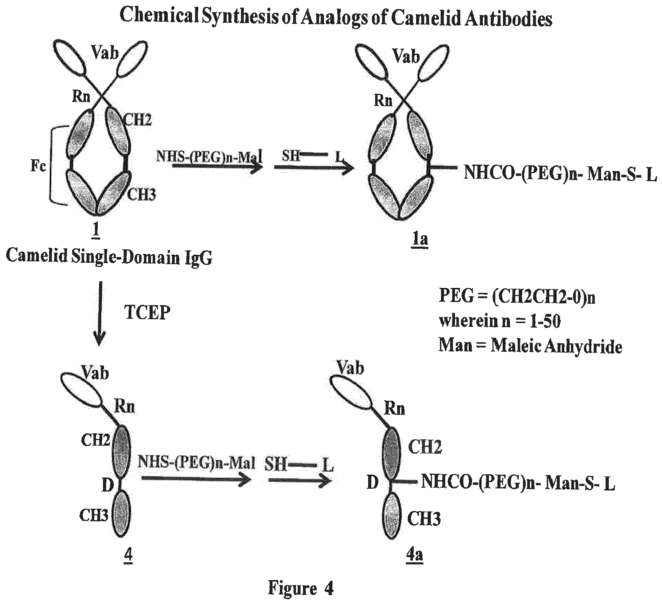

Schematics for derivatization and immobilization of mini-antibodies 1 are shown in figure 4. Mini-antibody 1 (1 mg, 11 nmols) will be treated with commercial NHS-(PEG)ₙ-Mal (11 ul of 10 mM stock= 110 nanomoles) wherein n= 1-50, in 50 mM MOPS/150 mM NaCl, pH 6.8, at RT for 1 hour to obtain the pegylated conjugate of figure 4 (structure not shown) which will be desalted by dialysis on C-3 Amicon filters to remove excess NHS-PEG-Mal reagent.

While the pegylation is underway, the ligand will be treated with 10X folds of Traut's Reagent in MOPS buffer, pH 6.8, containing 5% EDTA, at RT for 1 -2 hours. The thiolated ligand will then be purified either by dialysis (if ligands is chemical or biochemical entity) or by washing with MOPS buffer if ligand is a solid matrix.

The pegylated intermediate will be immediately conjugated with 10-20 folds excess of thiolated ligand: "SH- L" in MOPS buffer, pH 6.8 buffer containing 5 mM EDTA for 2-3 hours at room temperature (RT); where "L" may be enzyme (HRP, AP, Luciferase, galactosidase), protein, peptide, biotin, fluorophore, DNA, RNA, and solid matrix such as, magnetic beads, glass slides, gold nanoparticles, microchannels, microfluidic device.

When the ligand is a chemical or biochemical entity, for example, fluorophore, biotin, enzyme, protein, etc, the purification of the conjugate will be done by reverse-phase C8 HPLC.

Nucleic acid conjugates of mini- and nano-antibodies 1-15a will also be prepared using their pegylated conjugates followed by treatment with the thiolated-DNA/RNA molecules of interest (Fig. 4).

When the ligand is a solid matrix, such as, magnetic beads, glass slide, microchannels, etc., which we will use to immobilize the camelid antibodies, all we need to do is to wash the excess reagent with the appropriate buffer.

The activity and the amount of camelid antibody loaded onto the solid matrix will be determined by ELISA and commercially available protein assay kits.

### Single-domain Heavy-Chain Camelid Micro-Antibody 4 and its Analogs 4a:

Micro-antibody, 4, will be prepared by treating mini-antibody 1 (2 mg) with 1.0 ml of 10 mM TCEP (tris-carboxyethyl-phosphine) in 20 mM Phosphate/150 mM NaCl, pH7.4 at room temperature (RT) for one hour. The resulting micro-antibody 4 will be desalted on centricon-3 to remove the excess reagent and the buffer and stored at 4°C in 1X PBS.

Derivatization of 4 into 4a will be accomplished by the method described above for conversion of 1 into 1a.

### Single-Domain Heavy-Chain Camelid Sub-nano-antibody 5 and its Analogs of Structure 5a:

Micro-antibody 4 will be treated with trypsin or pepsin under controlled conditions at RT to cleave the CH2-CH3 domains from the antibody. After deactivation of the proteolytic enzyme with fetal calf serum, the subnano-antibody 5 will be isolated using size exclusion chromatography.

Derivatization of 5a into 5a will be accomplished by the method described above for conversion of 1 into 1a.

### Single-Domain Heavy-Chain Camelid Nano-antibody 6 and its Analogs of Structure 6a:

Sub-nano-antibody 5 will be treated with pepsin at a low pH of 4.5 in 2M sodium acetate buffer under mild conditions for 1-8 hours to cleave the CH2-CH3 domains from the antibody. After deactivation of the proteolytic enzyme with fetal calf serum, the nano-antibody 6 will be isolated using size exclusion chromatography.

Derivatization of 6 into 6a will be accomplished by the method described above for conversion of 1 into 1a.

### Single-Domain Heavy-Chains Bivalent Nano-antibody 7 and its Analogs of Structure 7a:

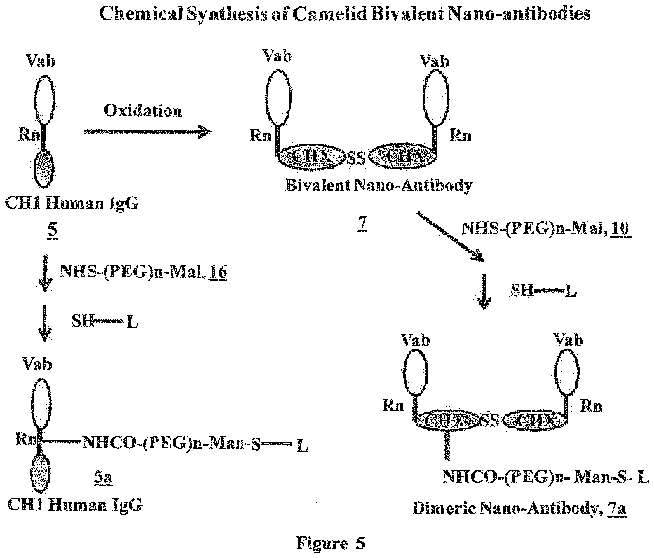

Schematics for the chemical synthesis of dimeric nano-antibodies and heir analogs are displayed in figure 5. Nano-antibody 5 will first be oxidized with 1% iodine in 20% tetrahydrofuran/70% water /10% pyridine for 5-10 minutes to transform into the dimeric nano-antibody 7. Treatment of 7 will be done with commercial NHS-(PEG)ₙ-Mal, wherein n= 1-50, in 50 mM MOPS/150 mM NaCl, pH 6.8, at RT for 1 hour to obtain the pegylated intermediate with maleimido group (structure not shown in figure 5) which, after purification by dialysis on C-3 Amicon filters, will be immediately conjugated with thiolated-ligand in MOP buffer containing 5% EDTA at pH6.8 for 2-3 hours at RT to obtain, after purification, 7a. The dimeric conjugate 7a will then be characterized by ELISA and Western blot assays.

### Trivalent and Tetravalent Camelid Nano-antibodies and Analogs:

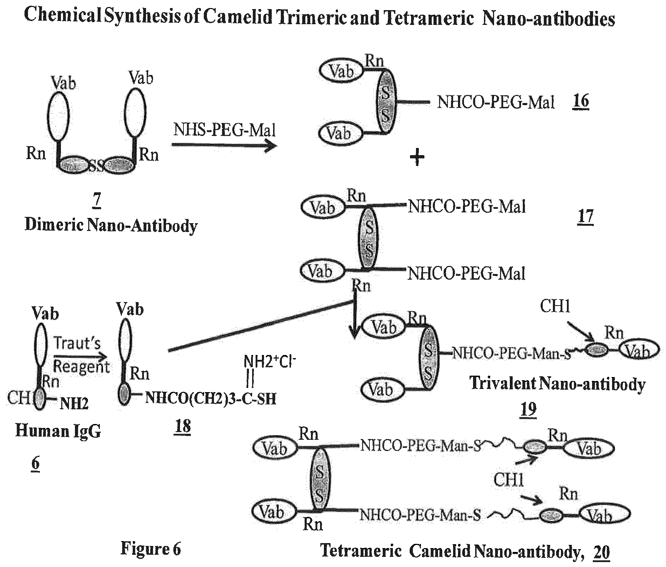

Schematics for the chemical synthesis of trivalent and tetravalent camelid nano-antibodies without the light-chains, and their analogs are shown in figure 6.

### Protocol for Developing Trivalent 8 and Tetravalent 9 Camelid Nano-antibodies:

Bivalent nano-antibody, 7, prepared by oxidative dimerization or chemical ligation, will be conjugated with NHS-(PEG)3-Mal (10 folds excess) in MOPS buffer at pH 7.0 for 1 hour at RT. Chemical ligation of the resulting monomeric and dimeric pegylated products 16 and 17 with the thiolated nano-antibody 18 (figure 6) will be carried out by combining the two at pH 6.8 buffer containing 5 mM EDTA and allowing the reaction to occur at RT for at least 2 hours. The so formed trivalent, 19, and tetravalent nano-antibody 20 will be purified by size exclusion chromatography and stored at 4°C in PBS containing 0.02% NaN3.

The attachment of a ligand to 19 and 20 can be readily done by making use of the lysine(s) of the hinge region to conjugate with the NHS-L.

Pentavalent and higher analogs of nano-antibodies (Vab domains of camel antibodies) can be similarly prepared.

### Production of Single-Domain Heavy-Chain Shark IgNAR (Structure 2):

Immunization of Sharks and Isolation of Shark IgNAR: Baby sharks will be immunized with the desired antigen(s), for example ALZAS, Tau, Aβ42 peptide which are the potential biomarkers for Alzheimer's disease, following the protocol described by Suran et al [J. Immunology, 99, 679 (1967)]. Briefly, the antigen (20ug per kg animal weight), dissolved in 20 mg/ml keyhole limpet hemocyanin (KLH) supplemented with 4 mg /ml complete Freund's adjuvant, will be injected intramuscularly. Four booster shots every two weeks four weeks after the initial injection will be administered.

After immunization, 3-5 ml shark blood will be withdrawn from the animal and the total IgGs will be precipitated out using 50% ammonium sulfate, followed by centrifugation at 2000 RPM for 10 minutes. After discarding supernatant, the precipitate will be dissolved in 20 mM PBS/150 mM NaCl containing 0.02% sodium azide and size fractionated on Sephadex G200. The conventional IgGs, MW ~230 KDa, will be separated out from the shark IgNAR with MW of ~180 K Da. Alternatively, the conventional IgG fraction will first be depleted with protein G bound to magnetic beads, followed by isolation of V-NAR protein with magnetic beads coated with protein-A. Affinity purification to obtain high affinity shark Ig-NAR, 2, will be done by magnetic beads coated with antigenic peptide.

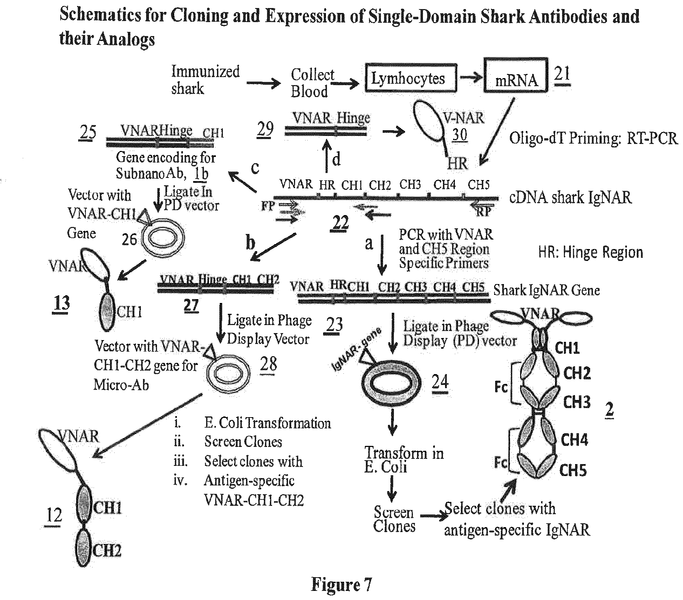

After determining the amino acid sequence of IgNAR, 2, nucleic acid sequence will be derived based from the amino acid sequence and recombinant DNA protocols will be established to produce the shark single-domain antibody 2 on a large scale. Schematics for cloning and expression of IgNAR are shown in figure 7.

### Isolation of RNA from Immunized Shark's Lymphocytes and Cloning:

Isolation of total RNA, 21, from immunized sharks will be done from 3-5 ml of shark blood using commercially available RNA extraction kits such as Bio-Rad's AquaPure®R RNA Isolation kit. Reverse transcription using oligo-dT primer will be achieved by PCR using high fidelity DNA polymerase to obtain the IgNAR cDNA, 22, shown in Fig.7.

### Recombinant Production of Shark Heavy Chain Only Antibodies and Their Analogs

An exemplary cloning strategy is shown in Fig. 7. Amplicons for IgNAR cDNA, 22, and its analogs will be performed using the following protocol:
IgNAR cDNA = 1.0 ug
Primers Mix = 10 pmol (forward and reverse primers)
1 mM dTNPs = 10 ul
10 mM MgCl2 = 5 ul
10X PCR Buffer = 5 ul
Taq DNA Polymerase = 0.6 ul
Water to = 50 ul

After first denaturation round of 94°C for 10 minutes, 35 to 36 cycles of amplification will be performed under conditions as described below:
Denaturation: 20 seconds at 94°C
Annealing : 30 Seconds at 56°C
Extension: 50 seconds at 72°C
Final Extension: 10 min, 72°C

All or portions of IgNAR cDNA using different combinations of the following forward and reverse primers.
Forward primers
5'-gcatgggtag accaaacaccaag-3' (SEQ ID NO: 1)
5'-gcgtcctcagagagagtcccta-3' (SEQ ID NO: 2)
5'-gagacggacgaatcactgaccatc-3' (SEQ ID NO: 3)
5'- gggtagaccaaacaccaagaacagc-3' (SEQ ID NO: 4)
Reverse primers
5'-gttctagccaataggaacgtatag-3' (SEQ ID NO: 5)
5'-gtttgcacaagagagtagtctttac-3' (SEQ ID NO: 6)
5'-cctaaattgtcacagcgaatcatg-3' (SEQ ID NO: 7)
5'- gtgcagttccctagaagtcttg-3' (SEQ ID NO: 8)

After amplification, the amplicon will be purified on 1.5% agarose. The amplicon will be extracted from the gel and its 5'-end kinased with gamma-ATP for blunt-end ligation with the phage-display vector using T4 DNA-ligase following standard ligation protocols.

Library or Plasmid Construction: Prior to cloning, the PCR amplicon encoding IgNAR gene will be digested with Sfi1 and Not1 (Roche) following the cocktail:
V-NAR-CH1-CH2-CH3-CH4-CH5 DNA= 5 ug
10X Restriction Buffer 5 ul
Sfi1 (10 U/ul) 8 ul
Water to 50 ul
Incubate 50°C for 8 hour
Not1 35 U
Reaction Buffer 4.5
Water to 60 ul
Incubate at 37°C for 4-5 hours.
Ethanol Precipitate at -70°C Pellet
Water to 50 ul
Agarose gel (1.5%) purification
Pure DNA Encoding Shark IgNAR Antibody

### Vector Ligation:

IgNAR DNA = 200 ng
Vector DNA = 1000 ng
10X Ligase Buffer = 5 ul
T4 DNA Ligase = 10 U
Water to 50 ul
Incubate 15 hours at 4°C.
Ethanol Precipitate at -70°C
Suspend pellet in 10 ul.

### Electroporation:

250 ul of E. Coli TG1 cells will be made electrocompetent with BRL Cell-Porator® following vendor protocol.

Panning of Phage-Displayed IgNAR-Antibody 2 Library: Electroporated TG1 cells will be transfected with the phagemid-IgNAR DNA insert. Approximately, 1010 cells will be grown to mid-logarithmic phase before injection with M13K07 helper phages. Virions will be prepared as described in the literature [Andris-Widhopf J., et al, J. Immunology Methods, 242, 159 (2002)] and used for panning at a titer of 1013/ml. Specific IgNAR antibody against the antigenic peptide will be enriched by five consecutive rounds of panning using magnetic beads conjugated with antigenic peptide. Bound phage particles will be eluted with 100 mM TEA (pH 10.00), and immediately neutralized with 1M Tris.HCl (pH 7.2) and will be used to reinfect exponentially growing E. Coli TG1 cells.

The enrichment of phage particles carrying antigen-specific IgNAR antibody will be assessed by ELISA before and after five rounds of panning. After the fifth panning, individual colonies will be picked up to analyze the presence of the virion. binding by anti-M13-HRP conjugate.

### Expression and Purification of the Single -Domain IgNAR 2:

The selected positive clones will be used to infect a new bacterial strain, HB 2151, a non-suppressor strain that recognizes the amber codon as a stop codon for soluble protein production. The HB2151 cell harboring the recombinant phagemids will be grown at 28°C in 250 ml 2X YT-ampicillin, 1% glucose in culture flasks until OD600 0.7. The cells will be washed and resuspended in 250 ml 2X YT-ampicillin, supplemented with 1mM isopropyl beta D-thiogalactopyranoside (IPTG), and incubated over night at 22°C to induce protein expression.

Before adding IPTG to the cultures, a portion will be spotted on an LB/ampicillin plate for future analysis of the clones. The culture will be then be centrifuged at 4000 RPM for 15 minutes to pellet the bacterial cells. The culture supernatant will then be screened by ELISA for antigen-specific IgNAR protein 2.

### Chemical Synthesis of Single-Domain Heavy-Chain Shark Only Antibodies and Their Analogs

### 2a, 11, 11 a, 12, 12a, 13, 13a, 14, 14a, 15, 15a:

### Derivatization of Shark IgNAR 2 to Obtain Analogs of Structure 2a:

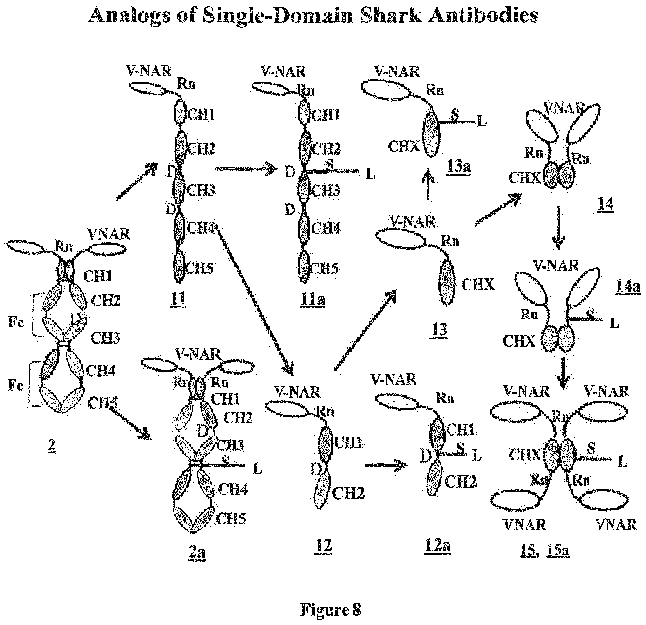

Schematics for derivatization of shark IgNAR 2 are shown in figure 8. Shark antibody 2 (1 mg, 6 nmols) will be treated with commercial NHS-(PEG)ₙ-Mal (6 ul of 10 mM stock= 60 nanomoles) wherein n= 1-50, in 50 mM MOPS/150 mM NaCl, pH 6.8, at RT for 1 hour to obtain the pegylated conjugate (structure not shown) which will be desalted by dialysis on C-3 Amicon filters to remove excess NHS-PEG-Mal reagent.

While the pegylation is underway, the ligand will be treated with 10X folds of Traut's Reagent in MOPS buffer, pH 6.8, containing 5% EDTA, at RT for 1 -2 hours. The thiolated ligand will then be purified either by dialysis (if ligands is a chemical or biochemical entity) or by washing with MOPS buffer if ligand is a solid matrix.

The pegylated intermediate will be immediately conjugated with 4-5 folds excess of thiolated ligand: "SH- L" in MOPS buffer, pH 6.8 buffer containing 5 mM EDTA for 2-3 hours at room temperature (RT); where "L" may be enzyme (HRP, AP, Luciferase, galactosidase), protein, peptide, biotin, fluorophore, DNA, RNA, and solid matrix such as, magnetic beads, glass slides, gold nanoparticles, microchannels, microfluidic device.

When the ligand is a chemical or biochemical entity, for example, fluorophore, biotin, enzyme, protein, etc, the purification of the conjugate 2a will be done by reverse-phase C8 HPLC.

Nucleic acid conjugates of shark IgNAR 2 and analogs 11,12,13,14, and 15 will also be prepared using their pegylated conjugates followed by treatment with the thiolated-DNA/RNA molecules of interest.

When the ligand is a solid matrix, such as, magnetic beads, glass slide, microchannels, etc., which we will use to immobilize the camelid antibodies, all we need to do is to wash the excess reagent with the appropriate buffer.

The activity and the amount of single-domain shark antibody loaded onto the solid matrix will be determined by ELISA and commercially available protein assay kits.

### Single-domain Heavy-Chain Shark Mini-Antibody 11 and its Analogs 11a:

Mini-antibody, 11, will be prepared by treating the IgNAR 2 (2 mg) with 1.0 ml of 10 mM TCEP (tris-carboxyethyl-phosphine) in 20 mM Phosphate/150 mM NaCl, pH7.4 at room temperature (RT) for one hour. The resulting micro-antibody 11 will be desalted on centricon-3 to remove the excess reagent and the buffer and stored at 4°C in 1X PBS.

Derivatization of 11 into 11a will be accomplished by the method described above for conversion of 2 into 2a.

### Single-Domain Heavy-Chain Shark Micro-antibody 12 and its Analogs of Structure 12a:

Mini--antibody 11 will be treated with trypsin or pepsin under controlled conditions at RT to cleave the CH3-CH4-CH5 domains from the antibody. After deactivation of the proteolytic enzyme with fetal calf serum, the shark micro-antibody 12 will be isolated using size exclusion chromatography.

Derivatization of 12 into 12a will be accomplished by the method described above for conversion of 2 into 2a.

### Single-Domain Heavy-Chain Shark Sub-nano-antibody 13 and its Analogs of Structure 13a:

Micro--antibody 12 will be treated with trypsin or pepsin under controlled conditions at RT to cleave the CH2 domain from the antibody. After deactivation of the proteolytic enzyme with fetal calf serum, the shark sub-nano-antibody 13 will be isolated using size exclusion chromatography.

Derivatization of 13 into 13a will be accomplished by the method described above for conversion of 2 into 2a.

### Single-Domain Heavy-Chain Shark Dimeric -Nano-antibody 14 and its Analogs of Structure 14a:

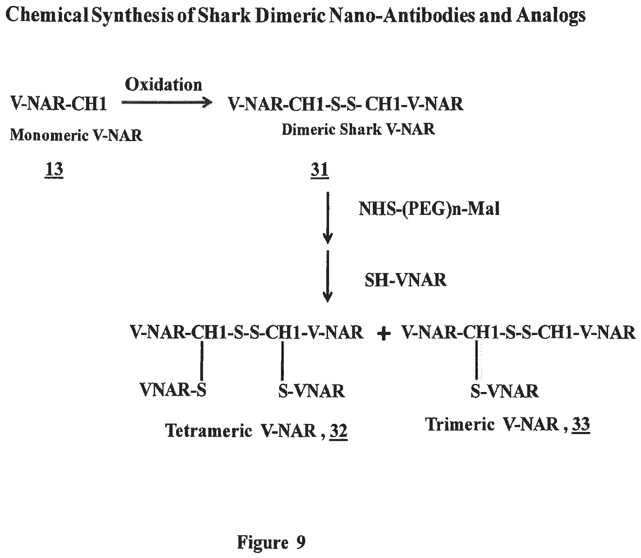

Dimeric V-NAR will be prepared by the oxidation of monomeric V-NAR, 13, to obtain 14 as described in figure 9. These protocols are general and do not need detailed explanation.

Likewise, the transformation of 14 into its analogs of structure 14a will be accomplished as described above for the preparation of 2a from 2.

### Tetrameric and Trimeric V-NAR Nano-antibodies 31 and 32:

Dimeric V-NAR nano-antibody 14 will be treated with 4-5 molar equivalent of NHS-PEG-Mal to obtain a mixture of tri- and tetra-pegylated derivatives of dimeric V-NAR nano-antibody (figure 9).

After purification, the tri- and tetrameric pegylated products will be treated with thiolated V-NAR to obtain, after purification by HPLC or just by dialysis, tetrameric and trimeric V-NAR nano-antibodies 31 and 32.

### Immobilization of Single-Domain Camelid Mini-Antibody and Shark IgNAR Antibody and Analogs onto Solid Matrixes:

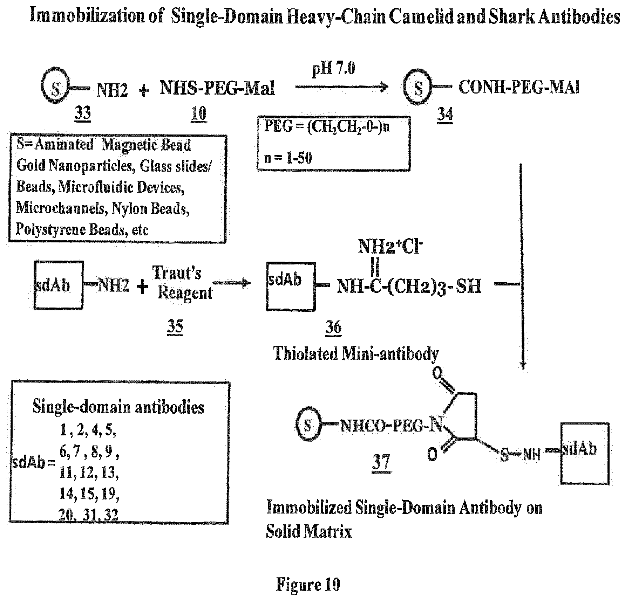

Immobilization of single-domain heavy-chain only shark and camelid native antibodies and their analogs onto solid matrixes, such as gold particles, magnetic particles, microchannels, glass particles and other solid surfaces will be accomplished using the steps outlined in Fig. 10. Aminated solid matrix 33 will first be derivatized with NHS-(PEG)n-Mal, 10, where n= 20 (20 fold molar excess) at pH 7.0 for 1 hour at RT. The solid matrix will then be washed thoroughly with the same buffer (50 mM MOPS/150 mM NaCl, pH 7.0). Any unconjugated amine groups will be masked with sulfoNHS-Acetate (Pierce) by incubated the solid matrix with 40 fold excess of the reagent at pH 7.0 for 60 minutes. After washing off the excess masking reagent, the pegylated matrix 34 will then be conjugated with thiolated single-domain heavy-chain antibody, 36, (10X excess) over the starting amine concentration. The conjugation will be performed at pH 6.5 for 2 hours at RT with gentle shaking of the matrix. The unused antibody will be recovered, and the matrix very well washed with 1X PBS /0.5% Tween-20 to obtain complex 37 in which nano-antibody is covalently bound to a solid matrix. The activity of the bound heavy-chain antibody will be measured using ELISA.

### Biomarkers for various diseases

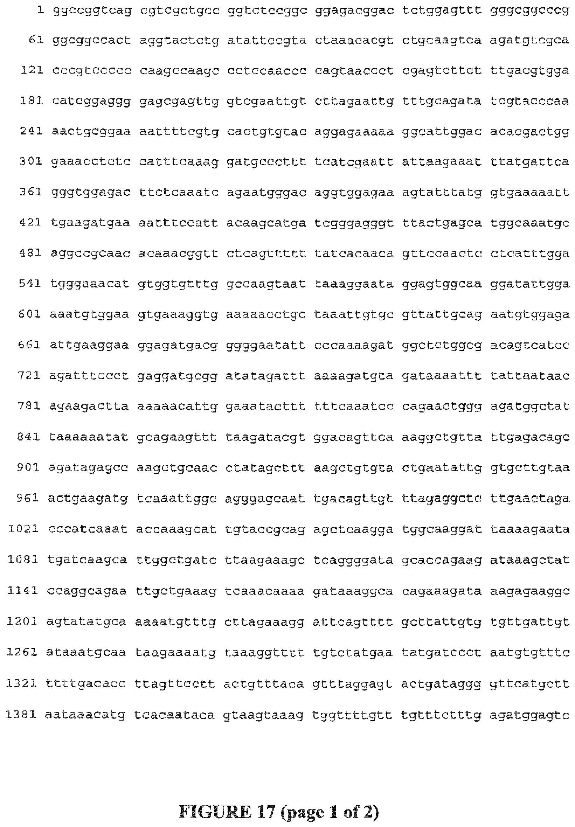

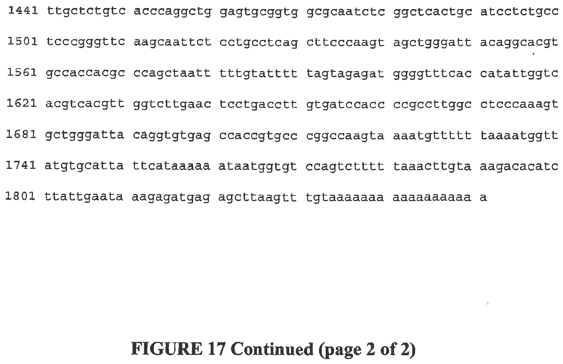

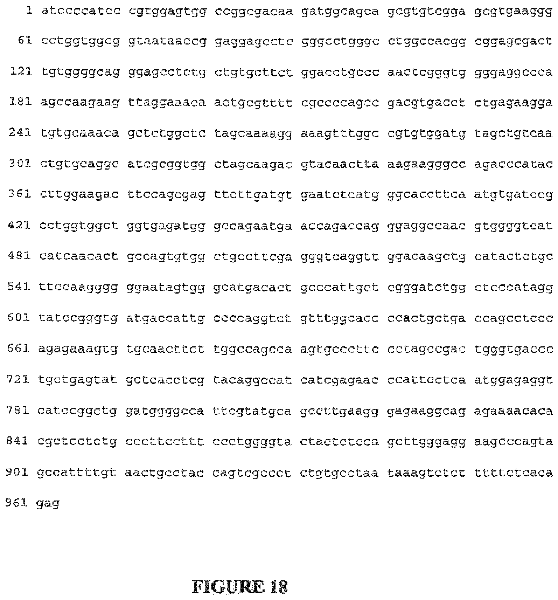

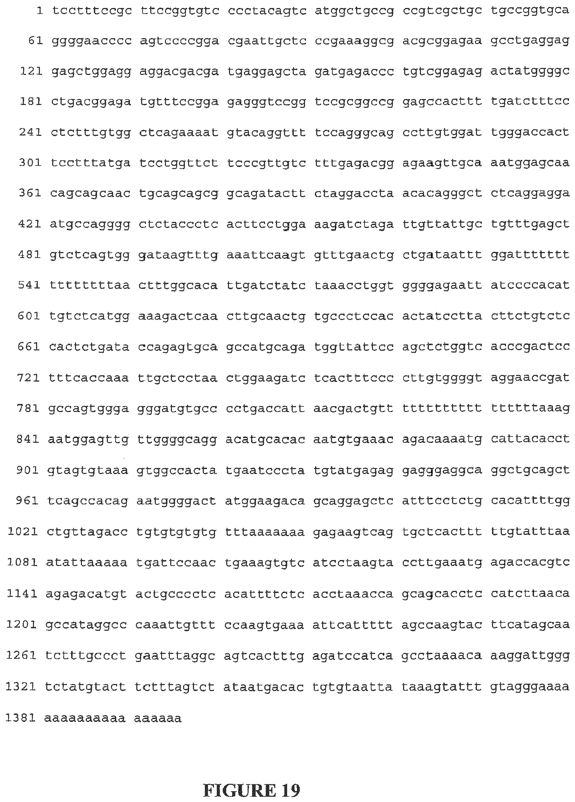

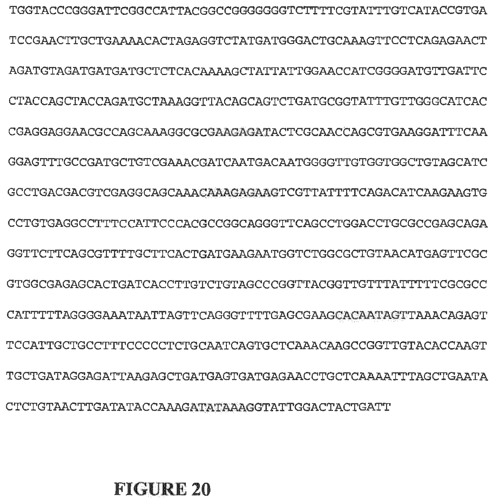

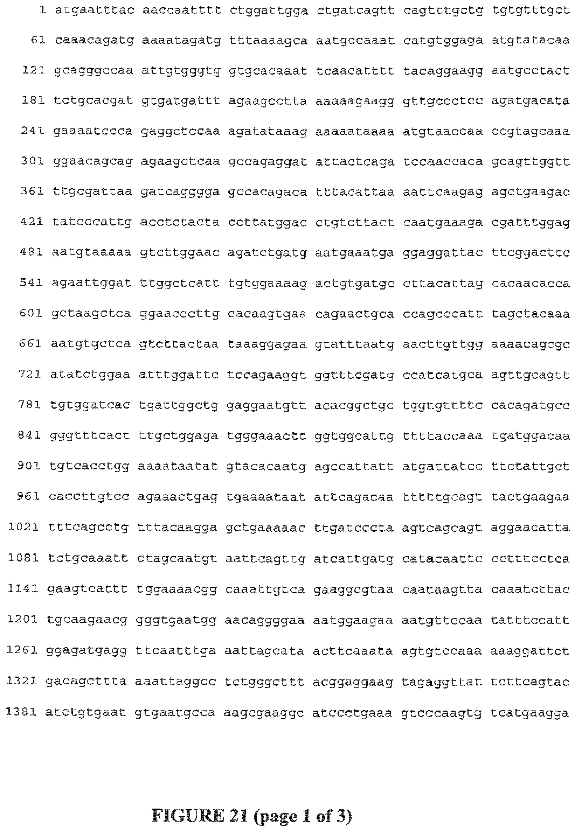

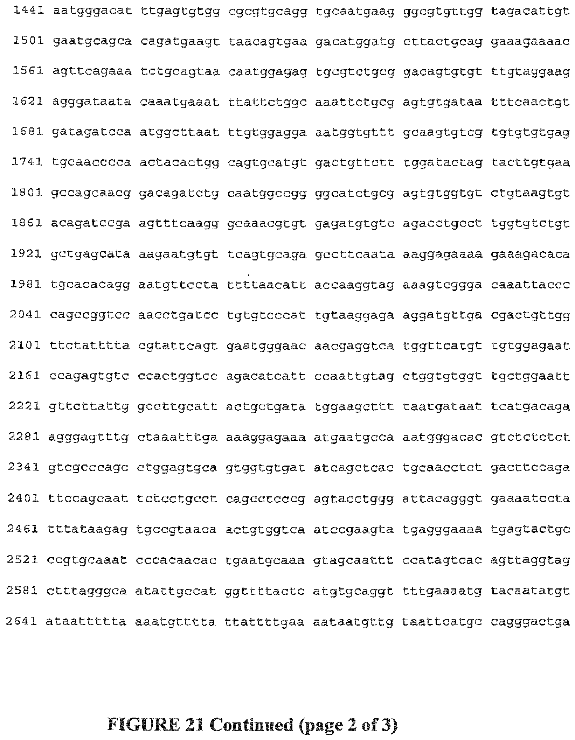

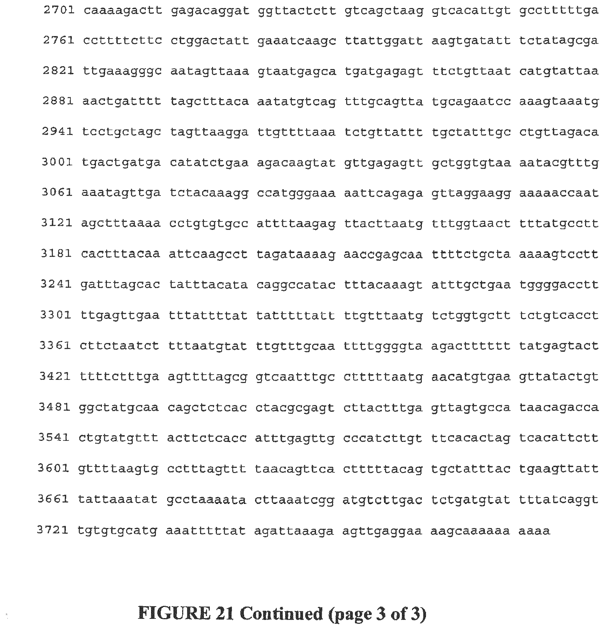

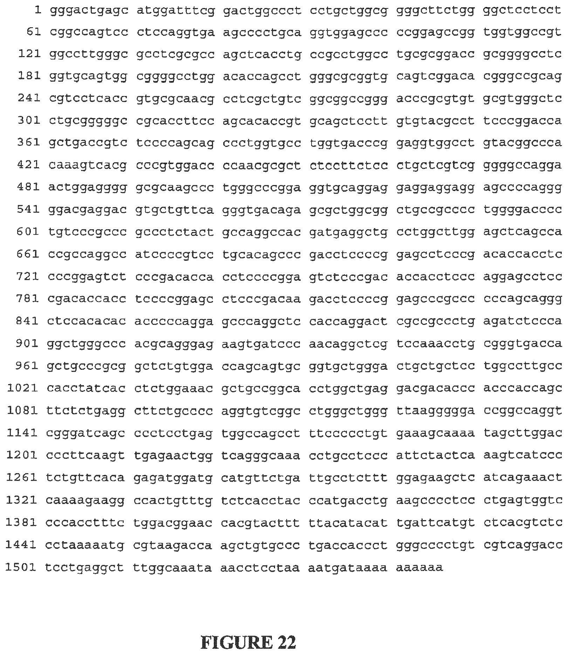

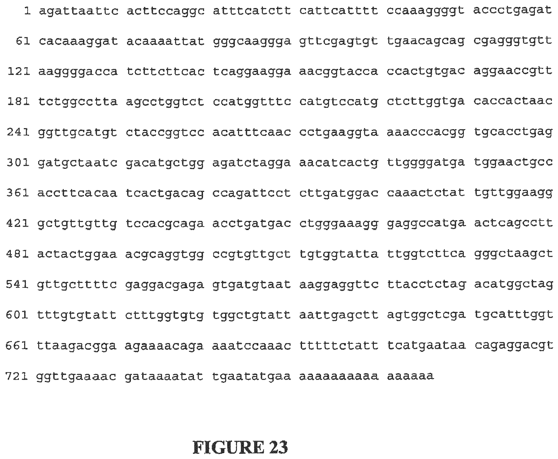

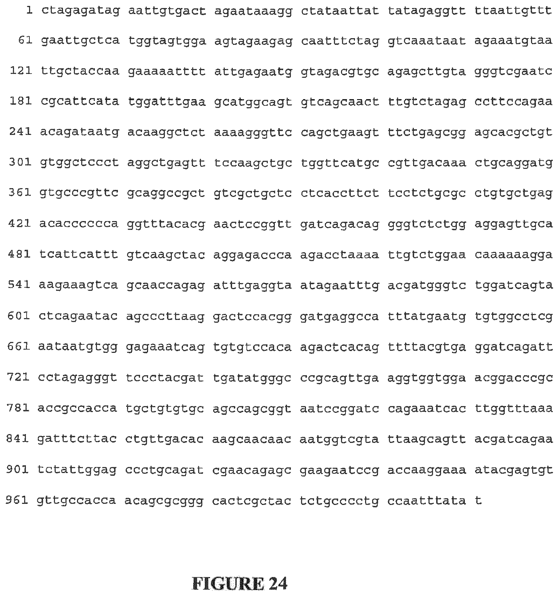

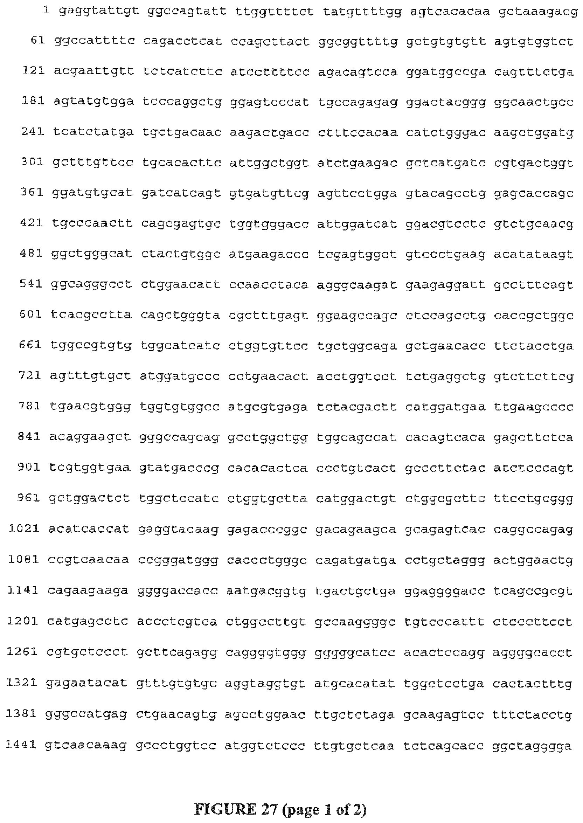

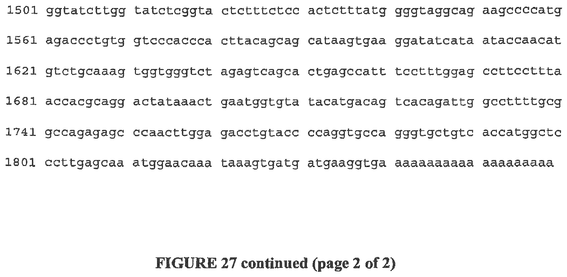

Single-domain heavy-chain only shark and camelid native antibodies and their analogs can be used to detect the presence or absence of one or more antigens or can be used for diagnosis of one or more diseases. Single-domain heavy-chain only shark and camelid native antibodies and their analogs can bind specifically to the antigens or one or more biomarkers for various diseases. Exemplary sequences of various biomarkers or antigens are disclosed in US application 12/563,330 filed September 21, 2009. Those sequences are incorporated by reference to its entirety. Exemplary sequences of nucleic acids encoding additional biomarkers for Alzheimer's Disease are disclosed in Fig. 17-27.

### Example 1:

### Capture and Detection of Pathogenic Antigens/Proteins Using Shark and Camel Single-Domain Antibodies (sdAbs)

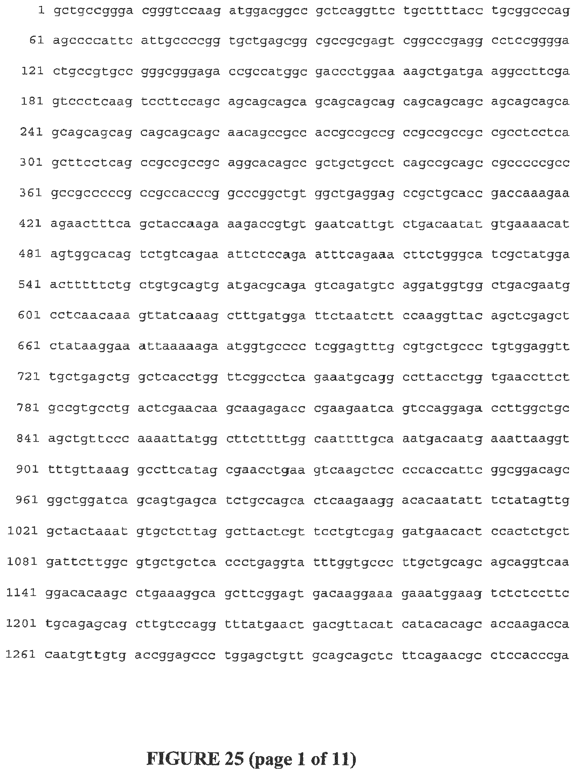

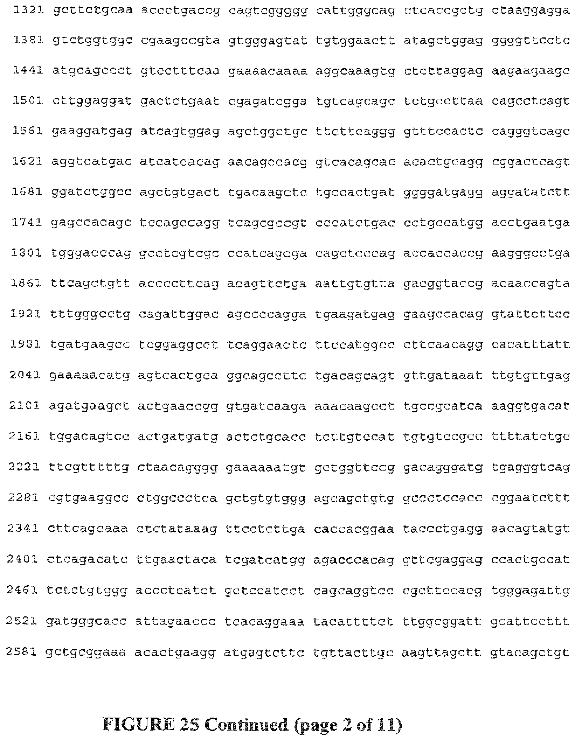

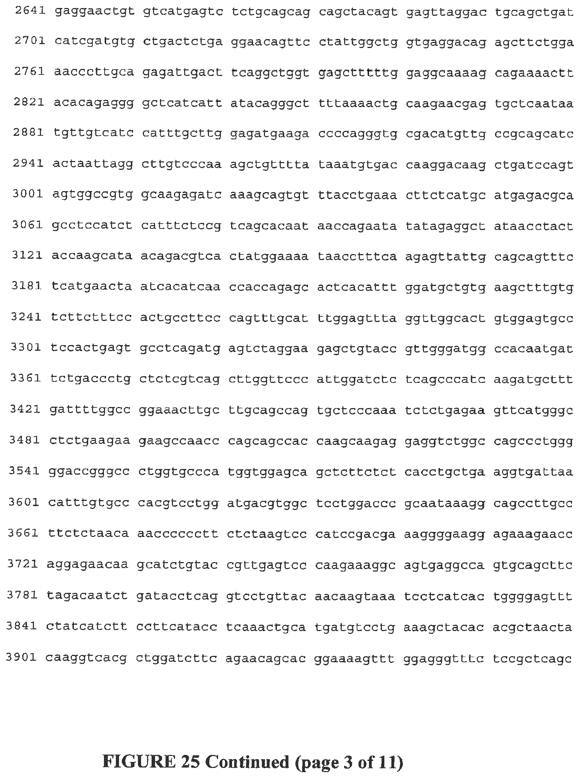

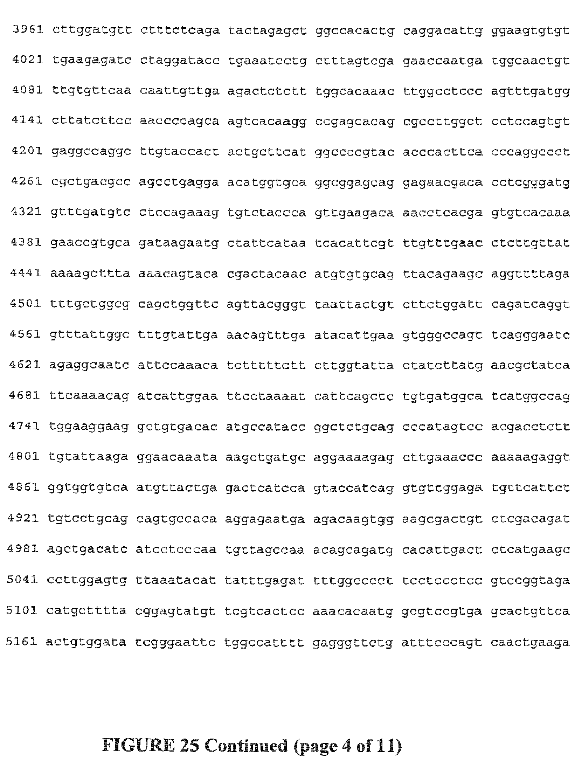

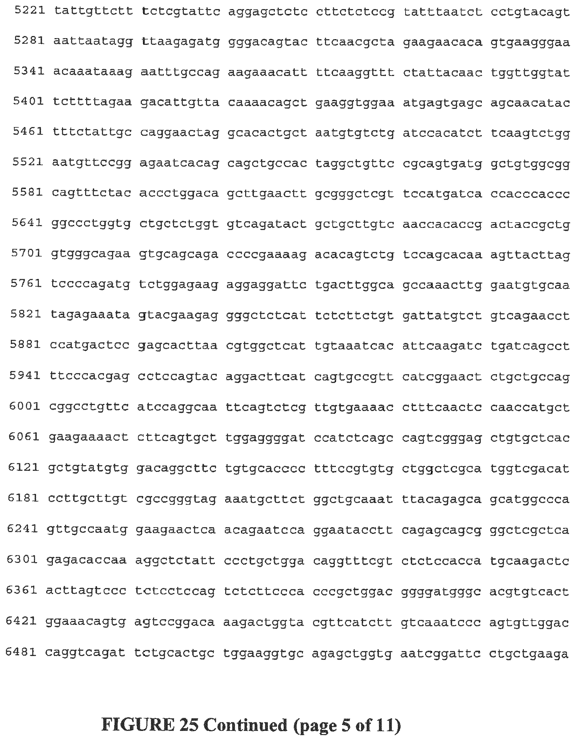

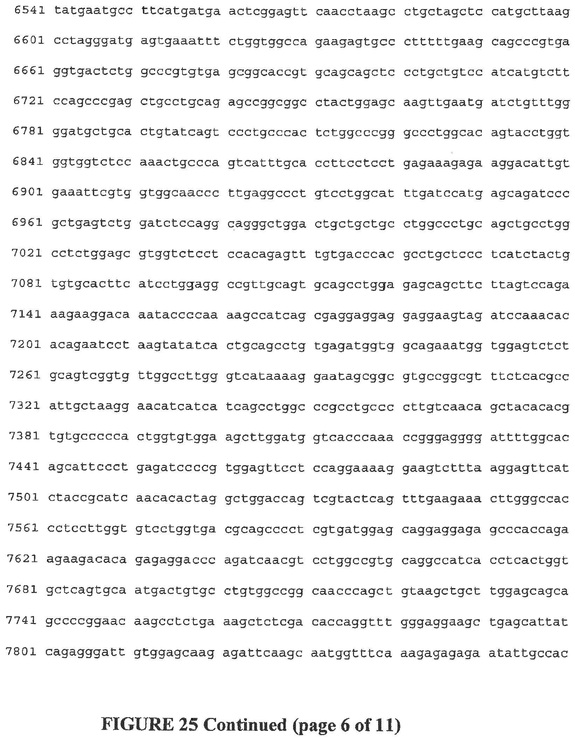

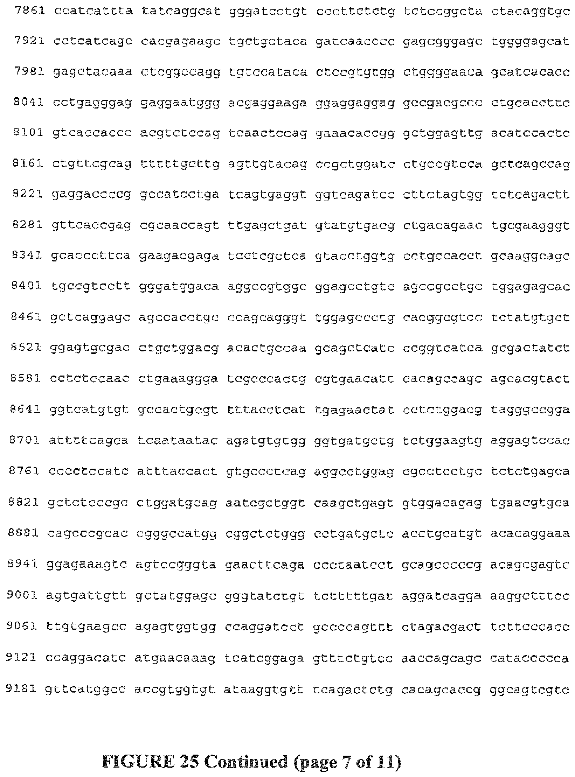

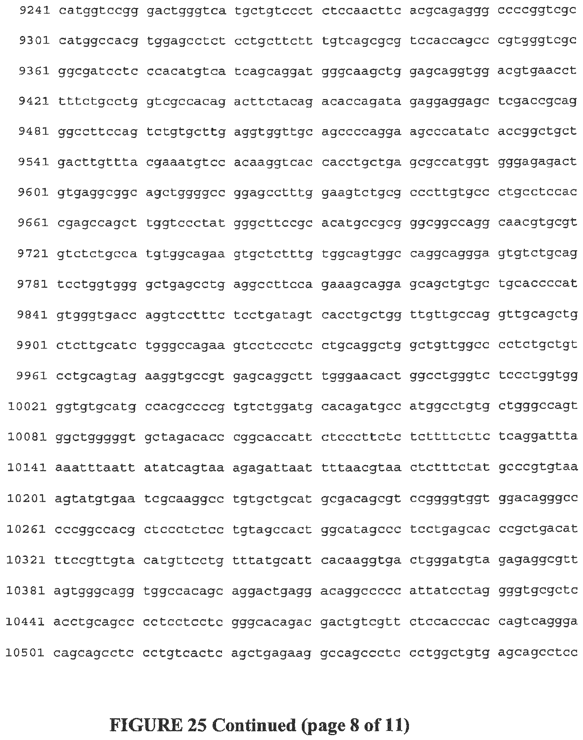

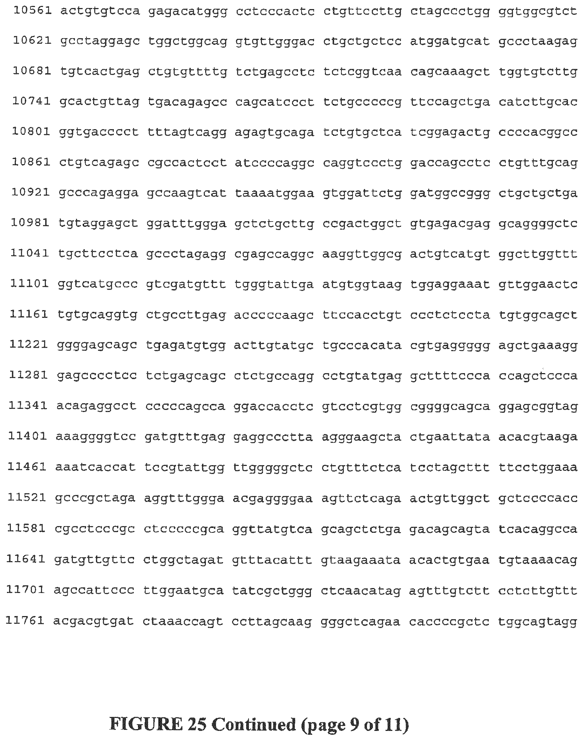

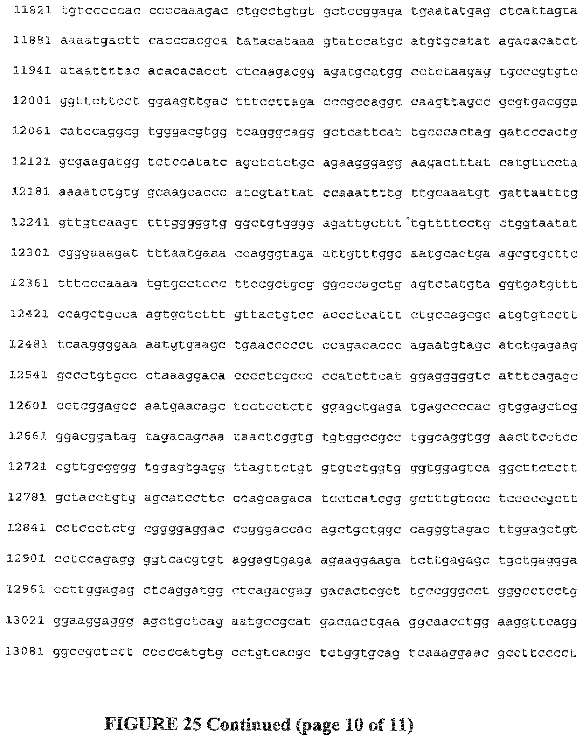

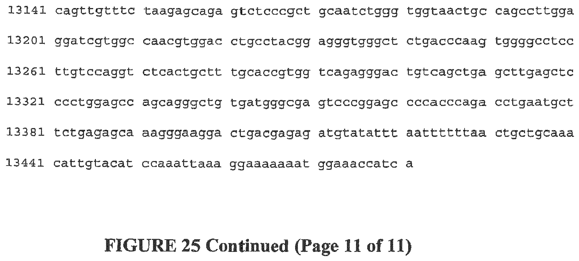

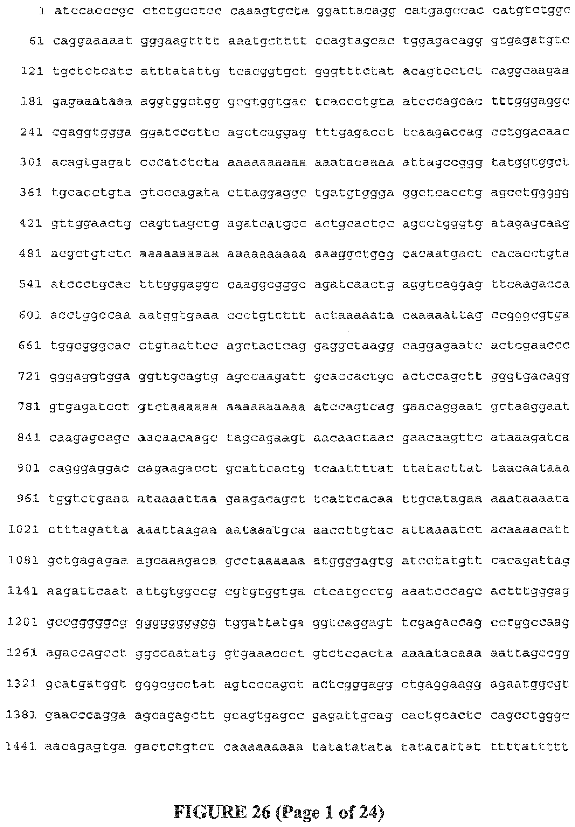

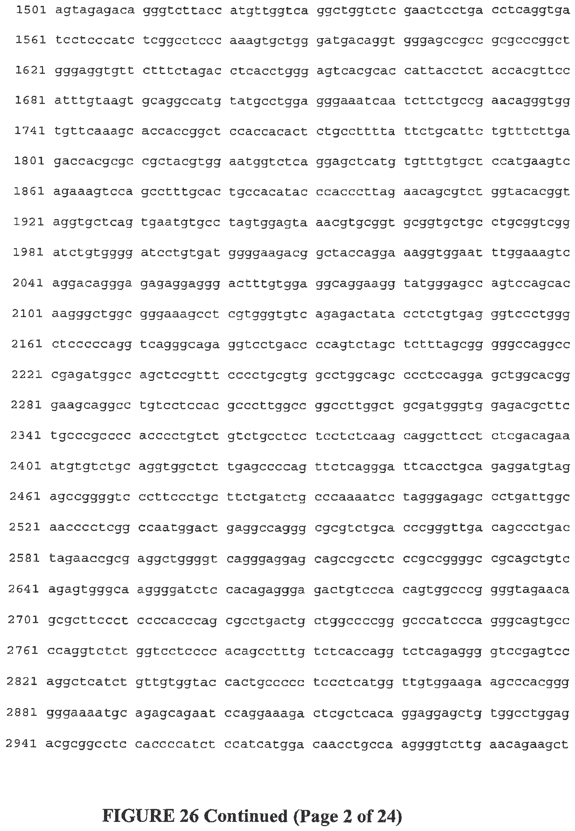

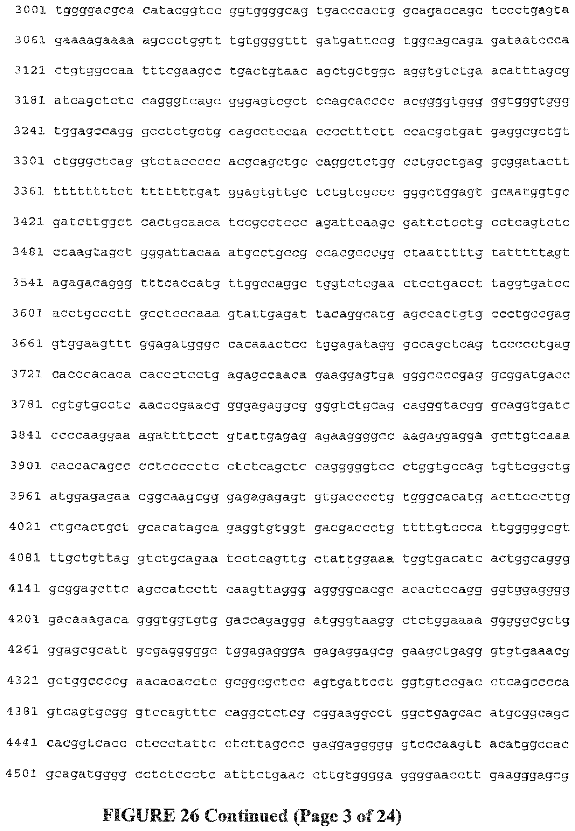

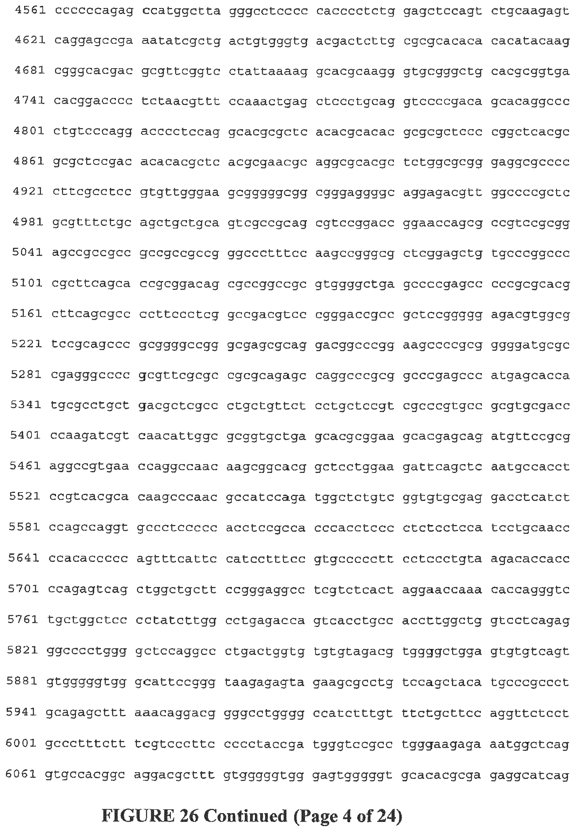

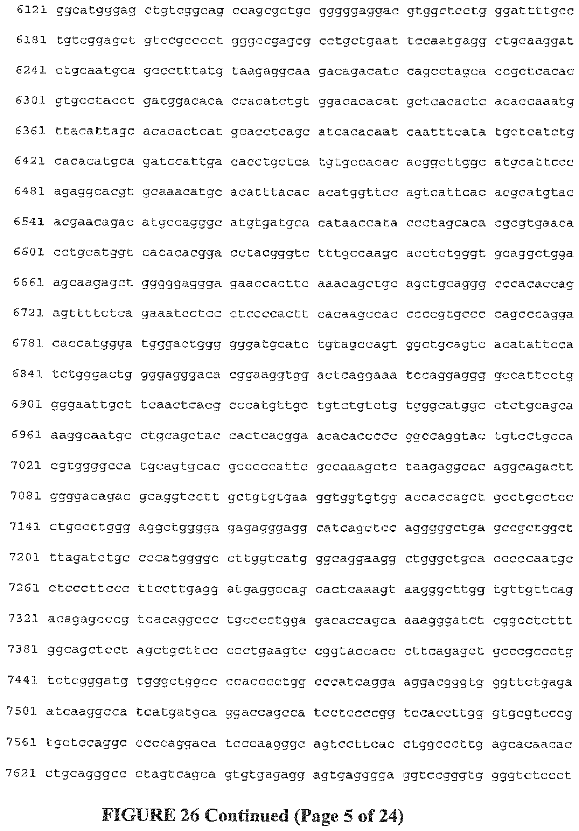

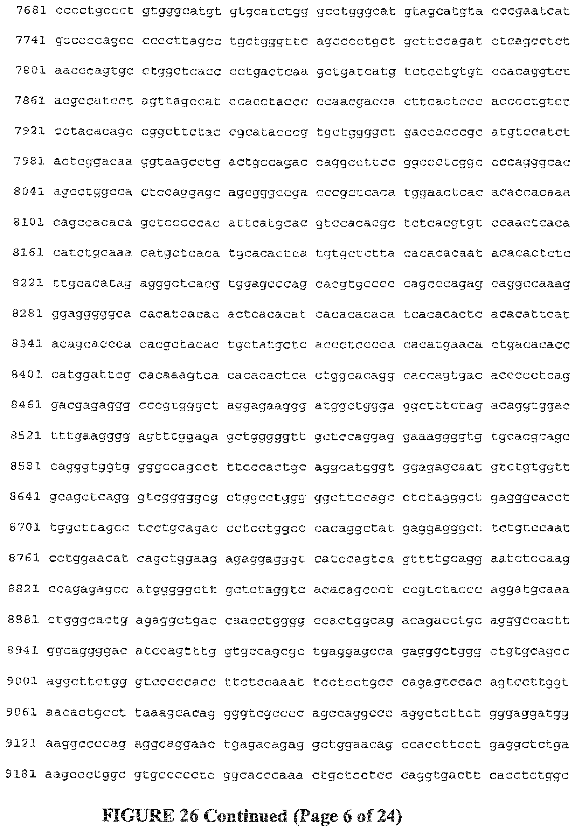

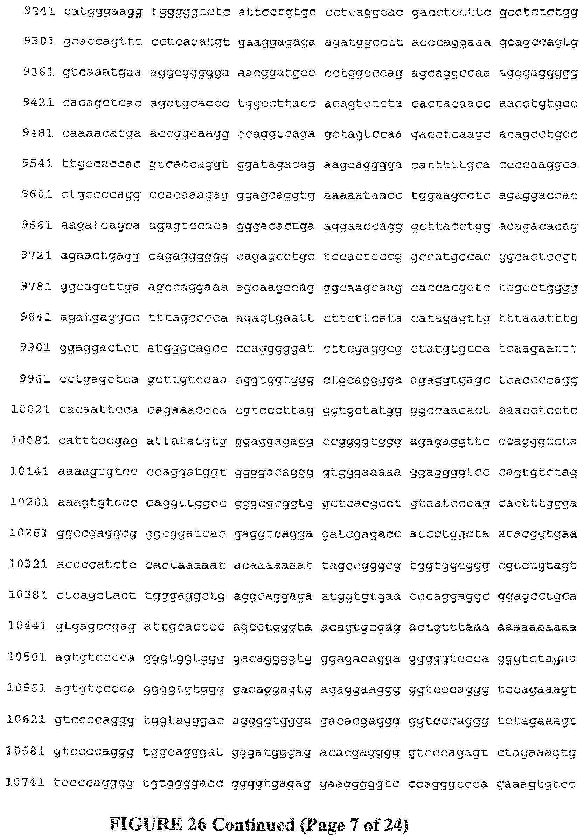

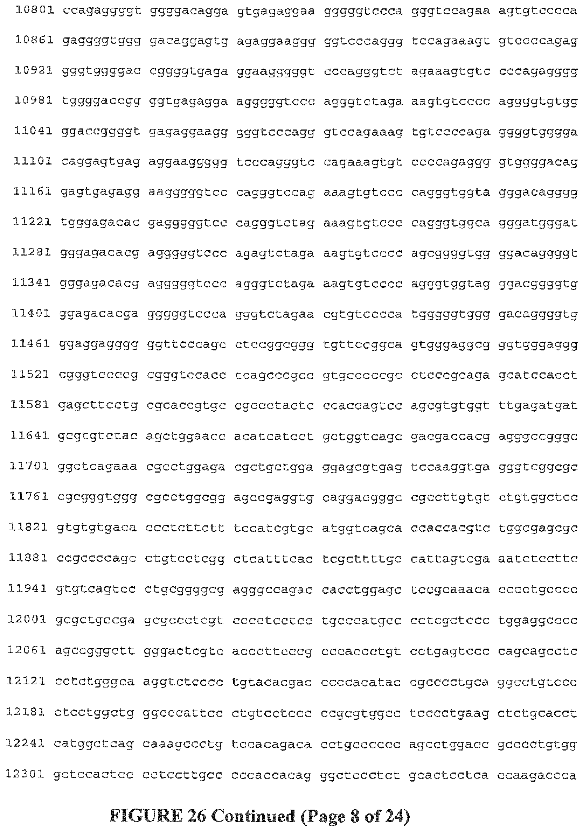

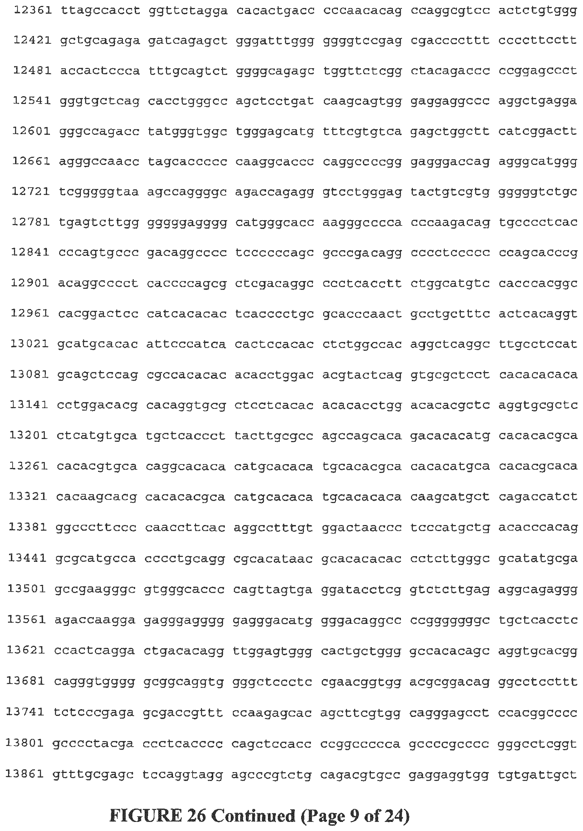

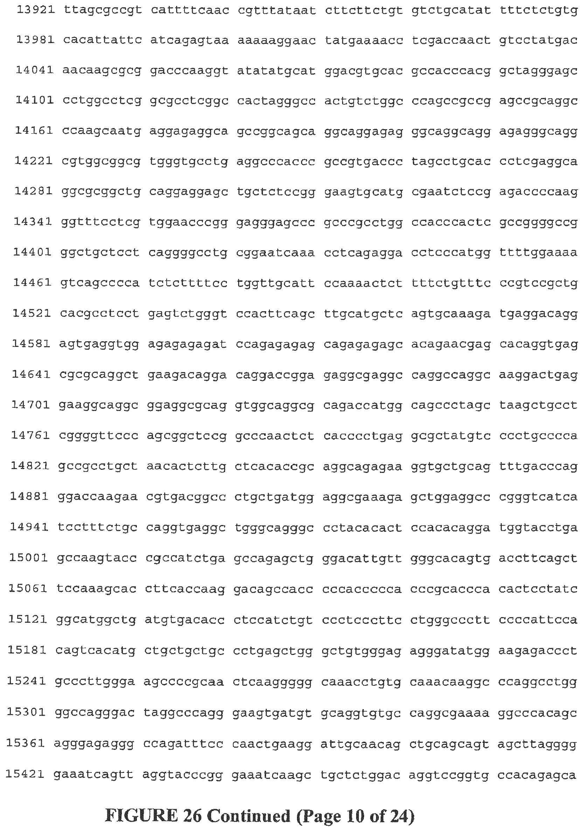

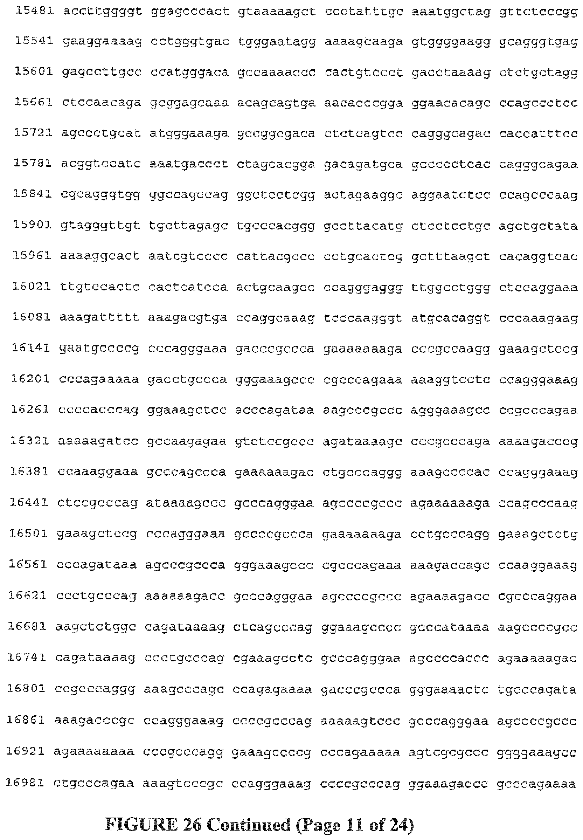

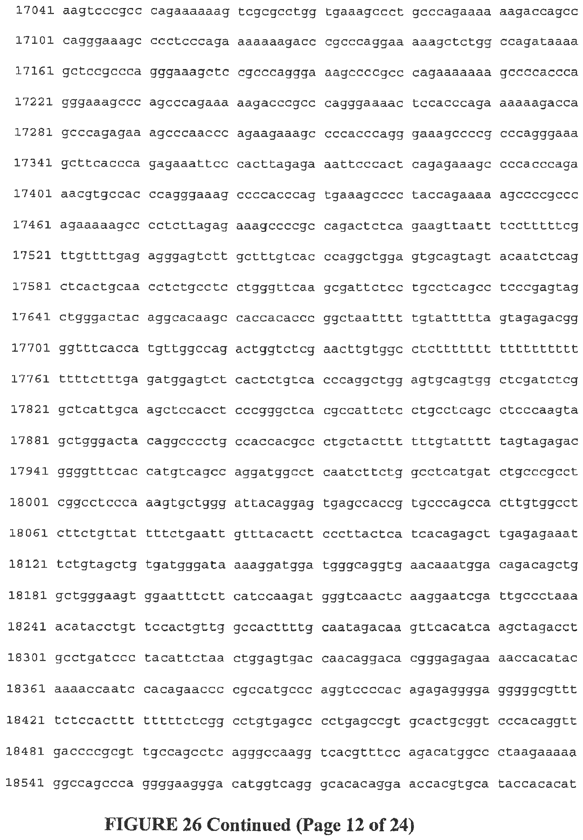

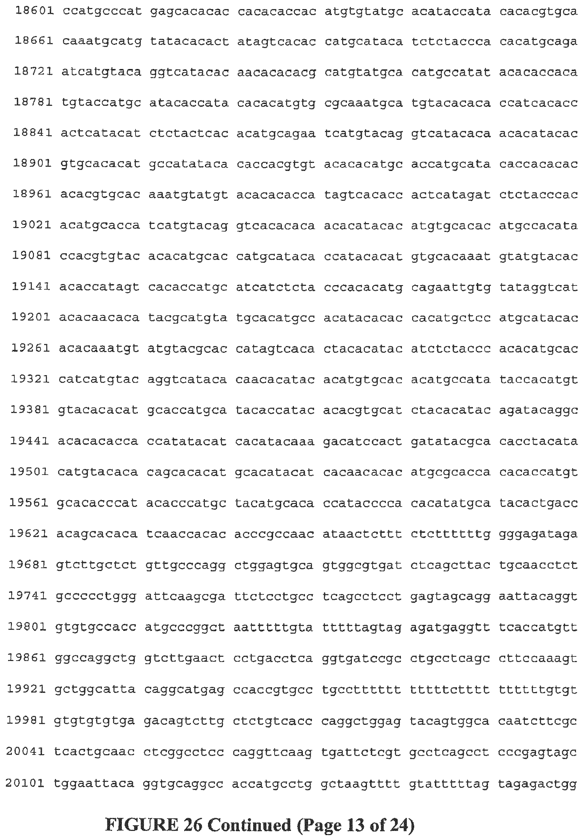

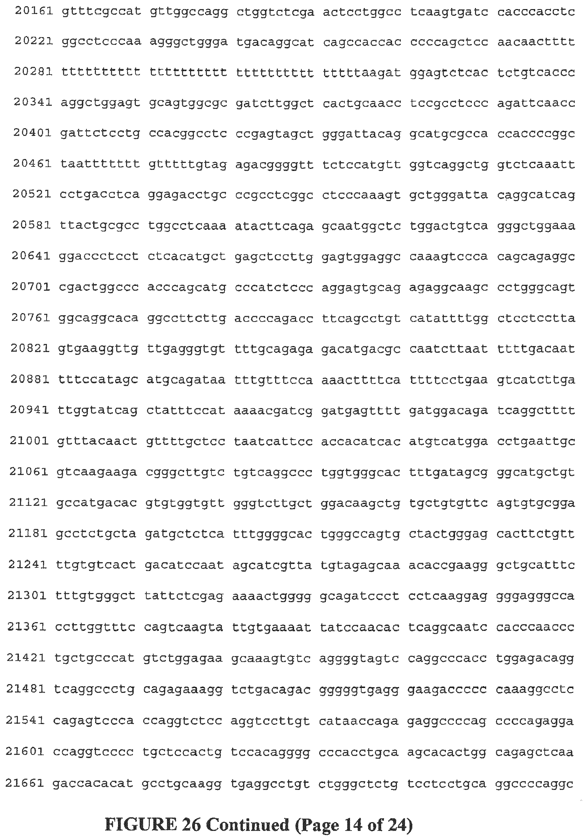

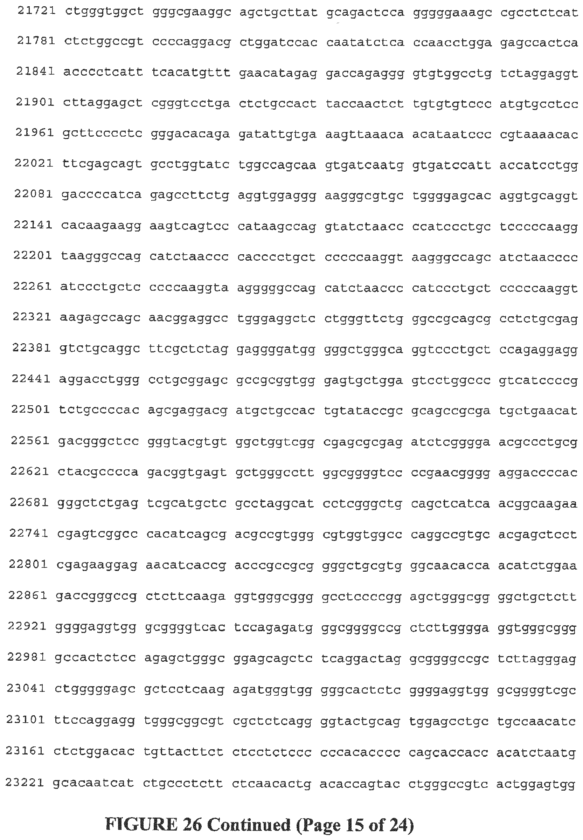

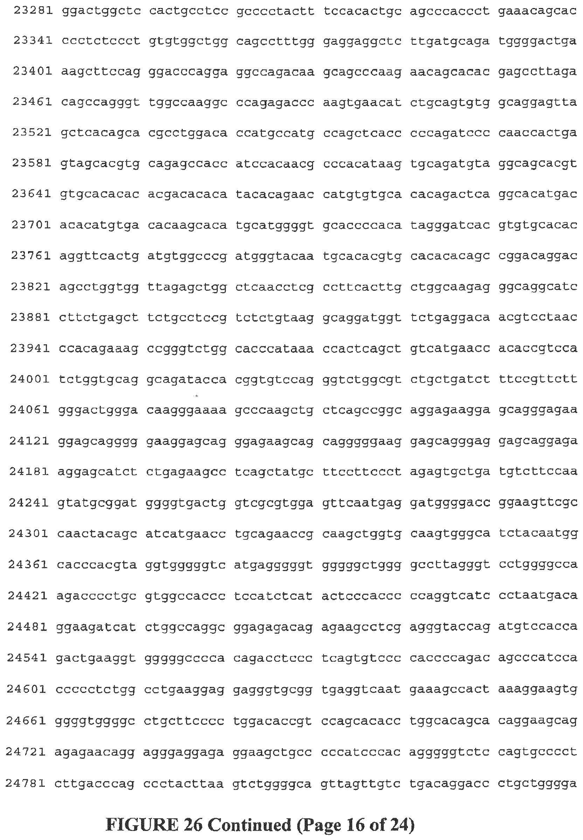

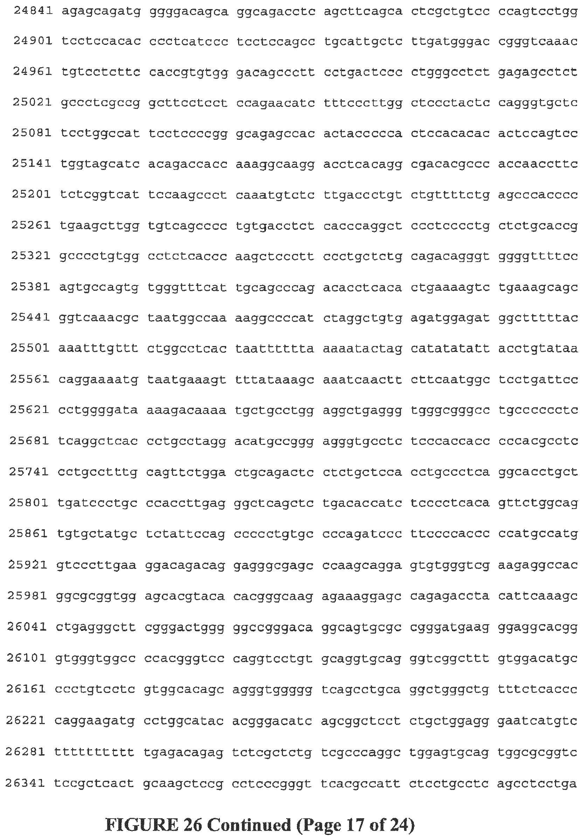

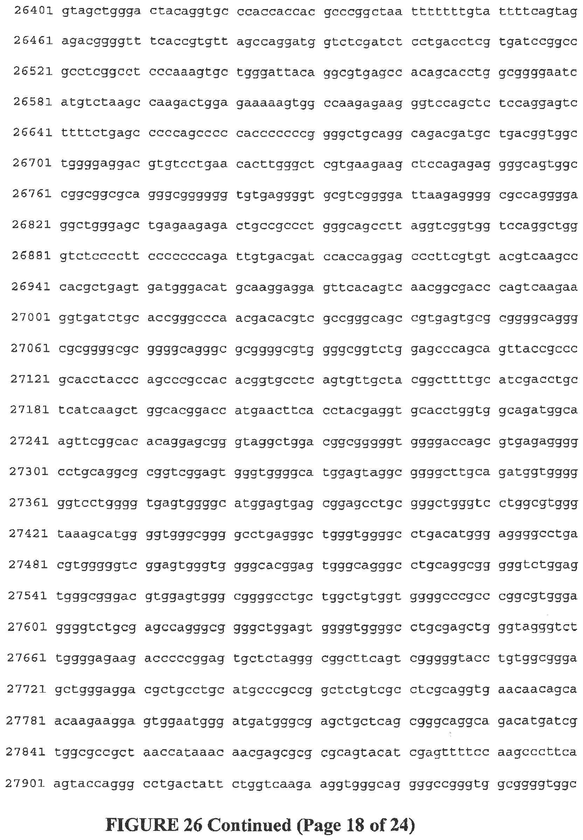

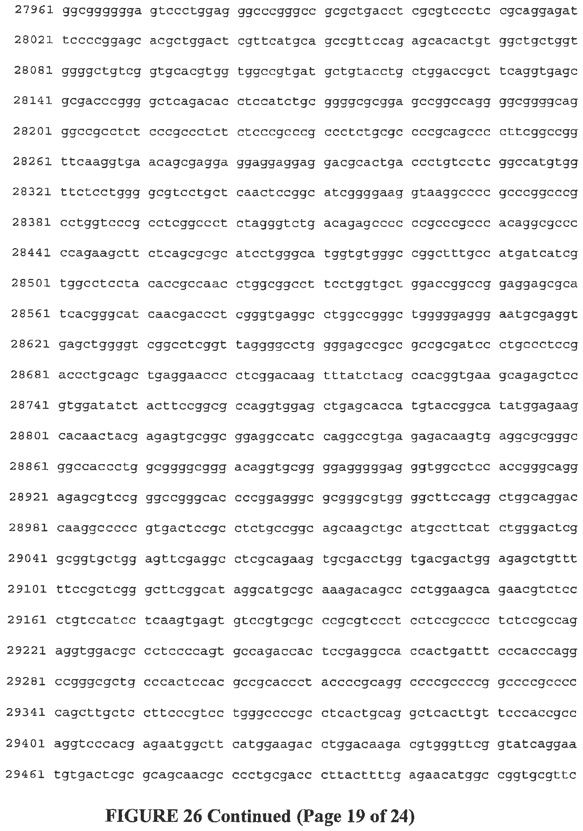

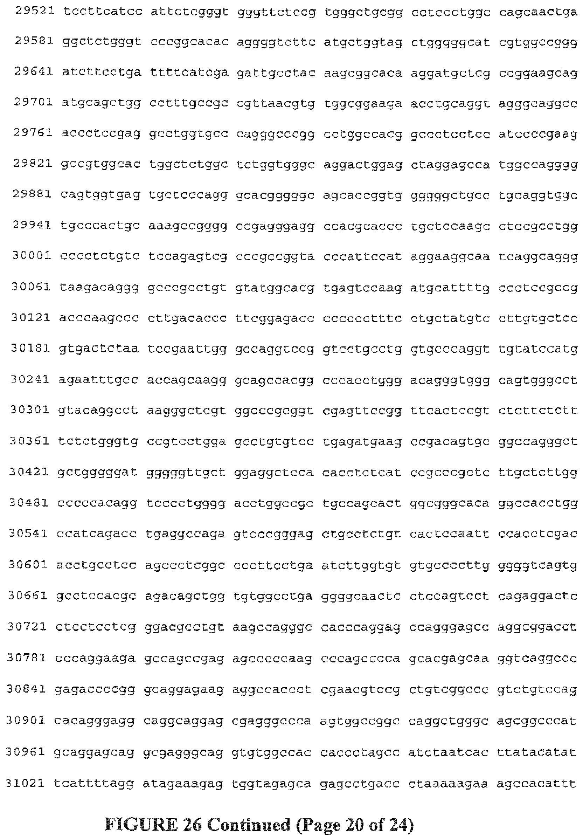

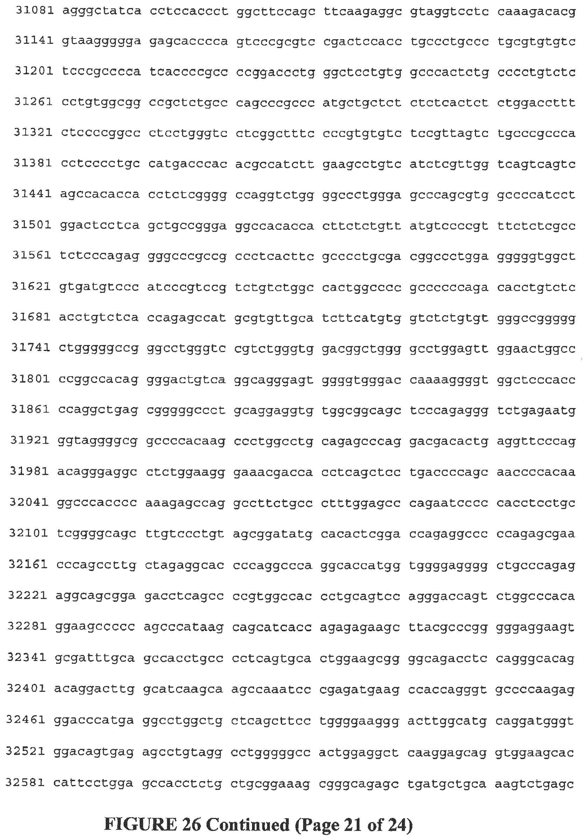

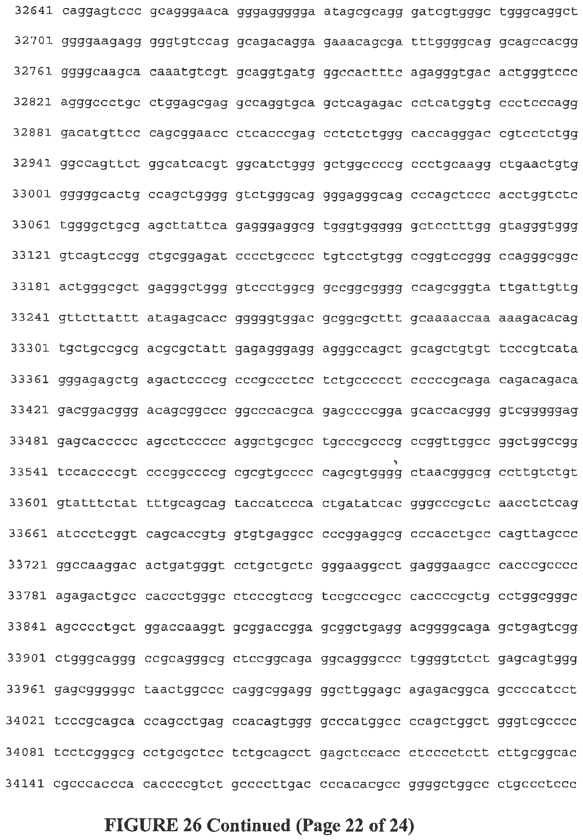

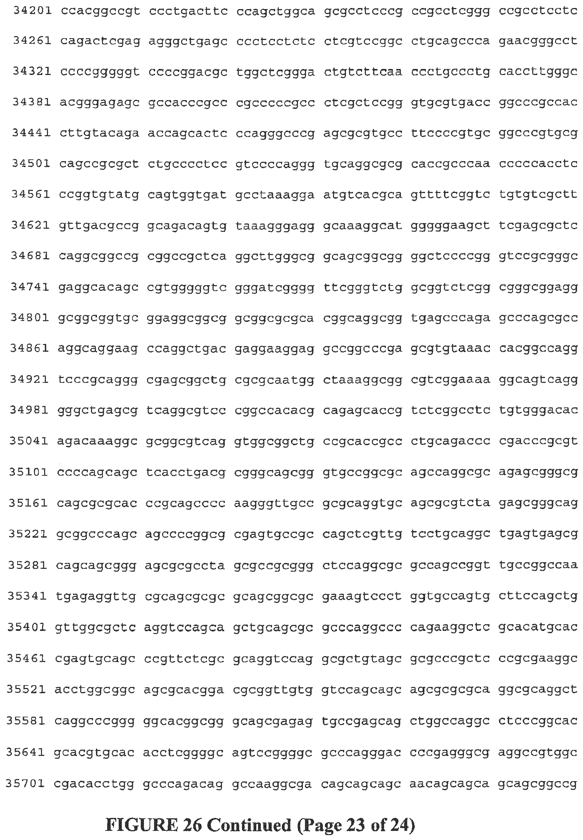

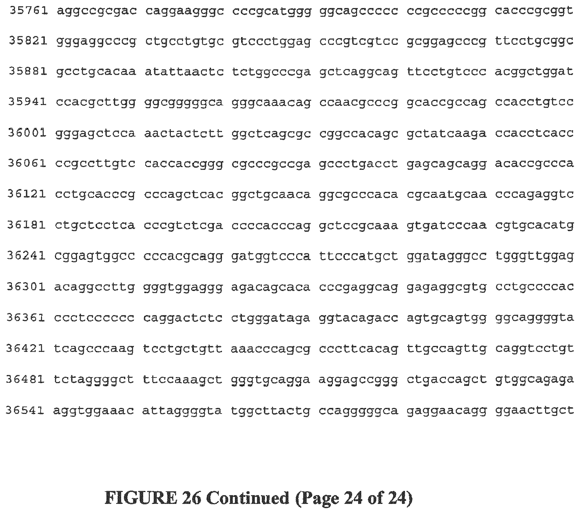

Serum from patient blood (10 ml), collected in EDTA tubes will be treated with shark and camelid heavy chain only antibodies and their analogs coated magnetic beads for 1-2 hours on a rotator with gentle rotation to bind the antigen. The beads will be separated using a magnetic rack and subsequently washed very well with PBS/1% BSA. The antigen-microantibody complex so formed will be treated with complex, detection antibody bound to an enzyme (AP, HRP, Luciferase, beta-galactosidase, gold particles) or DNA to sandwich the antigen between the shark and camelid heavy chain only antibodies and their analogs and the detection antibody forming the complex which will be detected either using an enzyme substrate or AgNO3 if the detection antibody is conjugated to gold particles. Exemplary schematics of the process is shown in Fig. 25. Alternatively, the detection antibody could be conjugated to DNA molecules which can then be amplified by PCR to obtain detection sensitivity equivalent to the detection of DNA by PCR as shown in Fig. 26.

### Example 2:

### Non-Invasive Detection of Prenatal Genetic Disorders from Captured Circulating Fetal Cells (CFCs) Using Heavy-Chain Antibodies

Blood (10 ml) from a pregnant woman will be treated at RT for 1 hour with sdAb conjugated to magnetic beads, with gentle shaking. The beads will be allowed to settle down in a magnetic rack and then subsequently washed with a wash buffer containing 20 mM PO4 -2/ 150 mM NaCl/0.1%Triton X-100 (3 x 2 ml) to ensure complete removal of blood and serum. The beads will then be washed with 1X PBS to remove triton. The bound DNA will then be eluted by hot 10 mM Tris.HCl, pH7.0 or by protease digestion.

This fetal DNA will then be analyzed by real-time PCR using Y-chromosome primers to test the gender and by chromosome 21 primers to test for Down syndrome.

### Example 3

### In-Vitro Capture of Pathological Proteins with Single-Domain Camelid and/or Shark antibodies and Detection by Enzymatic Signal Amplification:

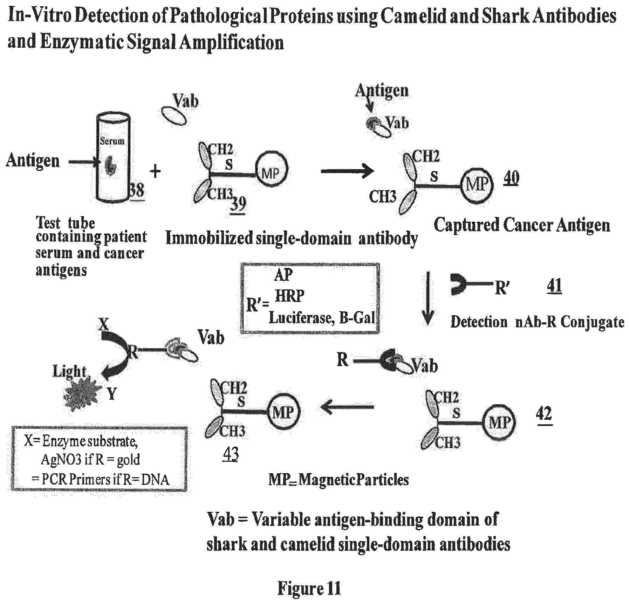

The high specificity of camelid and shark antibodies can be exploited to detect proteins at a much lower concentrations than what is currently possible. These antibodies are stable and functional at higher temperatures (80 to 90°C). Also, they are stable in the presence detergents and denaturing agents. This allows us to capture the pathological antigens under stringent conditions such as performing the capture reaction at elevated temperatures and using detergents (say for an example the use of up to 10% TritonX-100-), followed by high temperature stringent washings containing detergents to minimize non-specific capture. Such use of stringent conditions is only possible in immunoassays utilizing camelid and shark antibodies. When combined with enzymatic signal as shown in figure 11, the camelid and shark antibodies should be able to detect 0.1 to 1.0 attomoles of target molecules in 25 ul reaction volume, which itself is a much improvement over the existing proteomic detection technologies.

In the representative example shown in figure 11, the patient serum was incubated with magnetic beads coated with camel micro-antibody 39 (0.5 ml beads containing at least 1.0 ug camelid micro-antibody) with gentle shaking of the reaction contents at RT for 45 minutes. After 45 minutes, the beads were allowed to settle down and washed with 2XSSC buffer containing 1.0 % Tween-20. Detection of beads bound pathological antigen from 40 was accomplished by incubating the beads with a AP conjugate 41 of detection antibody for 1 hour at RT on a rocker. The beads were then thoroughly washed (5 x 2 ml) with preheated 2XSSC buffer (60 °C) containing 0.5% NP-40 to remove any non-specifically bound complex 41 (other commercially available detergents such as Triton X-100, Tween-20, SDS, LiDS, IGEPAL, Luviquat, DTPO, Antifoam 204, etc. can also be used.). The washings of the beads can also be done at temperature above 60 °C all the way up to 85 °C to remove any contaminants from the complex 42. The detection of complex 42 was then accomplished with Attophase (100 ul of 1.0 micromolar solution, 37 °C for 30 minutes), a fluorescence substrate for AP. The liberated green fluorescence was measured using a 96 microwell plate fluorimeter. 0.1 attomole of serum PSA antigen could be readily detected with 3:1 signal to background ratio.

### Example 4

### In-Vitro Capture of Pathogens by Single-Domain Shark IgNAR in Solution Phase and Detection by Enzymatic Signal Amplification:

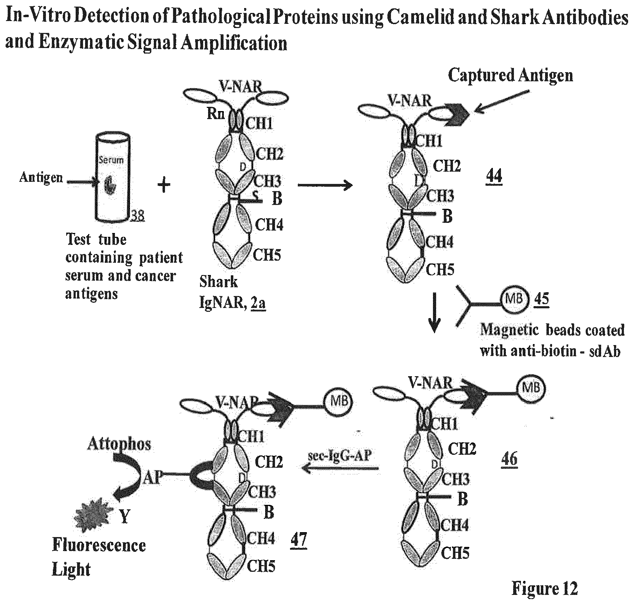

In this technology format, the biotinylated shark IgNAR, 2a (figure 12), can be added to the patient serum and allowed to react with the pathogen at 37°C for about one hour while the reactants are gently stirred or rotated on a orbital shaker. Anti-biotin camelid antibody (or shark antibody) bound to magnetic beads 45 can be added to reaction mixture to capture the so formed shark-IgNAR-Antigen complex 44 forming a complex of structure 46. The magnetic beads will be allowed to settle down in a magnetic rack and washed very well with a preheated (60 °C) wash buffer containing at least 1% NP-40. The complex 46 can then be detected by incubating with AP-IgG (sec) camelid complex using Attophos as a substrate as described above.

Other camelid and /or shark antibodies and their analogs can also be used the same way.

### Example 5

### Ultra-sensitive Signal Amplification Using Single-Domain Heavy-Chain Only Camelid and Shark Antibodies for In-Vitro Detection of Pathogens by Immuno-PCR:

The two vital components of the method of this invention are: #1) Ultra-specific capture of pathological proteins by the new generation of single-domain camelid and shark antibodies lacking the light-chains (heavy-chain only antibodies), and #2) an ultra-sensitive signal amplification technology to detect fewer than 200 molecules of protein biomarkers to diagnose diseases at a very early stage of the their manifestation. Therefore, in its preferred embodiment, this invention incorporates inherently highly specific heavy-chain antibodies for capturing the pathological proteins / antigens with high specificity with low to zero cross-reactivity, followed by detection of the captured antigen by enzymatic signal amplification, preferably immuno-PCR, to develop an ultra-sensitive, highly specific and reliable diagnostic assay to detect fewer than 200 copies of the pathological proteins from biological samples.

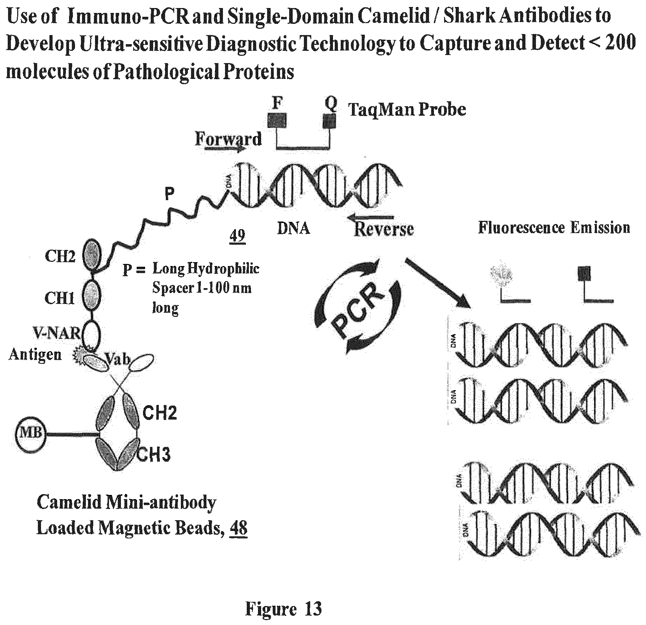

Figures 13 outlines the steps of the process involved. The protocol involves capturing the antigen from bodily fluid utilizing camelid and/or shark antibody coated magnetic beads by bringing in contact the said sample containing antigen with the beads. In the example shown in figure 13, camelid mini-antibody coated magnetic beads 48 are mixed with the serum for 1-2 hours with reactants constantly but slowly mixing all the time. The beads are then allowed to settle down in a magnetic rack and very well washed to ensure complete removal of the serum.

The detection of the captured antigen will be done by adding conjugate, 49, of secondary antibody that is conjugated to 100-120 bases long DNA via a hydrophilic linker that is at least 5 nanometer long to diminish and/or remove any stearic affects in the subsequent enzymatic amplification. The reaction between the captured antigen and the conjugate 49 will be allowed to take place for 2-3 hours after which the beads will be thoroughly washed to remove any unreacted conjugate 49.

The subsequent amplification of the attached DNA molecule by PCR using PCR kit from Applied Biosystems will allow for the indirect detection of the antigen with sensitivity almost equal to the sensitivity of detection of DNA by PCR.

There are many possible permutations and combinations of this technology. For instance, the antigen can be detected in solution phase by biotinylated or digoxigenin labeled camelid or shark antibody as described above following the steps of figure outlined in figure 12. The antigen-antibody complex so formed can be immobilized onto some solid matrix using camelid or shark anti-biotin or anti-digoxigenin antibody. Detection can be done by Immuno-PCR by forming a complex of the immobilized antigen-camelid-antibody with the secondary antibody bound to DNA which can be amplified by PCR.

Alternatively, the detection antibody in figure 13 can be conjugated with camelid or shark anti-biotin Mini- or nano-antibody. Biotinylated DNA can be used as a detection agent which will be amplified by PCR as outlined in figure 13.

### Example 6

### In-Vitro Capture and Detection of Rare Cells Using Shark and Camelid Heavy Chain Only

### Antibodies and Their Analogs

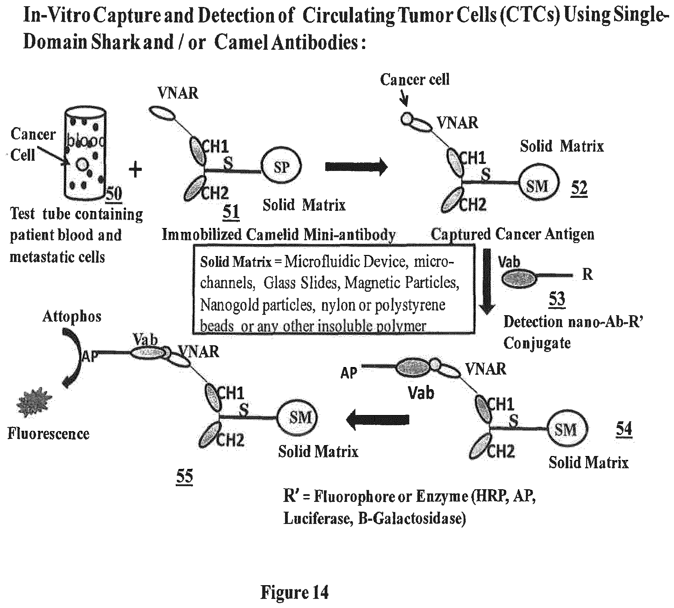

Fresh 5 ml patient blood will be diluted with 20 ml 1XPBS/1%BSA to 25 ml. To capture circulating tumor cells (CTCs), this sample will then be passed through a micro-fluidic device coated with an appropriate shark and camelid heavy chain only antibodies and their analogs, such as, anti-EpCAM-micro-antibody (camelid) 51 following flow rate recommended by the manufacturer of microfluidic device. To ensure that antibodies or its analogs do not lose any activity upon conjugation, all solid matrixes will first be coated with a hydrophilic polymer, such as, NHS-PEG-Mal (MW ~5000). The conjugation of the thiolated shark and camelid heavy chain only antibodies and their analogs with maleimido-group of the polymer can be achieved at pH 6.8 in a buffer containing 5% EDTA. Exemplary schematics of the process are shown in Fig. 14.

Alternatively, magnetic beads coated with EpCAM can be used. EpCAM (epithelial cell adhesion molecules) is frequently over expressed by carcinomas of lung, colorectal, breast, prostate, head and neck, liver, and is absent from hematological cells. The captured cells can be washed with 1% PBS (no BSA). The cell can be fixed with methanol, and then DAPI stained following CK8 or CK18 and CD45. Identification and enumeration will be done by fluorescence microscopy based upon the morphological characteristics, cell size, shape, and nuclear size. DAPI+, CK+, and CD45-cells will be classified as CTCs.

### Alternative Strategies to Capture Circulating Tumor Cells (CTCs):

Patient's blood (2-3 ml) (or urine 15-20 ml after centrifugation to pellet down the cells and suspending them in 1-2 ml HBSS media) will be incubated with an appropriate biotinylated mini-sdAb (1.5 ug/ml blood sample) at RT for one hour. For example, to capture epithelial cancer cells, such as from breast, prostate, and ovarian cancers, biotinylated- anti-EpCAM-mini-antibody (camel antibody against EpCAM antigens) will be used to label the circulating cancer cells in the blood. After diluting with HBSS or RPMI-1640 media or 1XPBS/2.5%BSA to lower the sample viscosity, the diluted blood is then passed through a microfluidic device coated with antibiotin-mini-camelid or shark antibody at a flow rate allowing maximum cell capture. The captured CTCs can then be fixed by fixing with methanol, followed by fixing with 1% PFA using any standard cell fixing procedures. Enumeration will then be done by DAPI staining followed by immunohistochemical staining with commonly used mouse mHCAb such as CK-7 but more preferably mini-CK-7 for higher specificity. CTCs have to be CD45 negative.

Alternatively, most of the RBCs from the blood sample can be first lysed using ammonium chloride solution (155 mM NH4Cl / 10 mM NaHCO3). After pelleting, the washed cells will be suspended in HBSS media (1-2 ml) and passed through the microfluidic device coated with heavy-chain antibody specific for the cell type one needs to capture and analyze.

Alternatively, the diluted blood sample after incubation with the biotinylated-antiEpCAM-mini-antibody or micro-antibody will be treated with the anti-biotin-mini-camelid antibody coated magnetic particles (Miltenyl) for 30 minutes while the sample is being gently rotated on a rotating wheel. After pulling down the magnetic particles with a magnet, the CTCs bound to the particles will be washed with PBS/1% BSA. The CTCs can then be enumerated by spreading them in a unilayer on a glass slide, drying them for one to two hours, followed by fixing with methanol, 1% PFA and staining the CTCs with CK-7.

Furthermore, these captured CTCs can be analyzed for the gene expression. For example, in case of prostate cancer patient, one can look for TMPRSS2-ERG translocation using PCR primers. TMPRSS2-ERG transcript is present in about 50% of the prostate cancer patients. Similarly, one can look for HER-2 expression in case of breast cancer.

### Example 7

### Capture and Analysis of Fetal Cells:

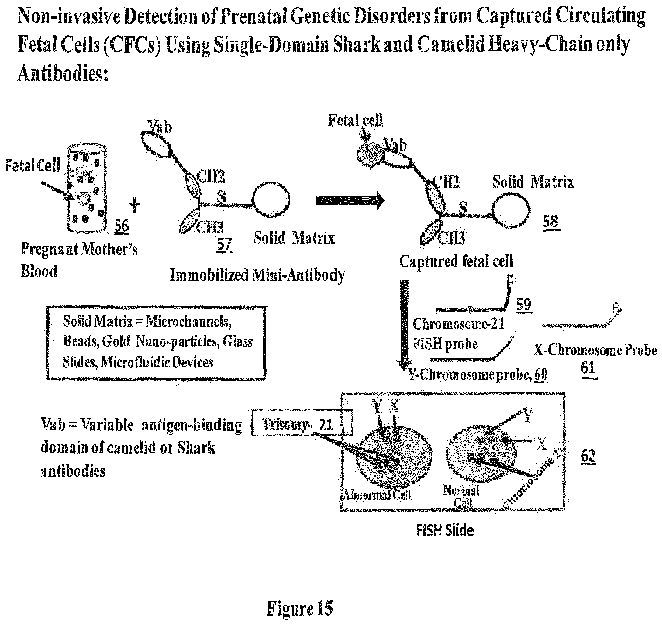

To capture fetal cells from the blood of pregnant mothers, 5 ml blood from pregnant mothers can be diluted to 15 ml with HAM-F 12 media containing 1% BSA and passed through the microfluidic device coated with the camelid and/or shark antibodies against the fetal cell surface antigens, CD71, glycophorin-A (GPA), CD133, and CD34. The captured fetal cells be analyzed for fetal gender, and genetic abnormalities using either PCR but preferably FISH probes for chromosomes X, Y, 13, 18 and 21 as shown in figure 15. For FISH analysis, the captured cells will be fixed with methanol followed by fixation with 1% PFA. After staining with epsilon-hemoglobulin, the cells be hybridized with Vysis FISH Fetal male gender can be readily detected by the appearance of XY fluorescence signal under the fluorescence microscope. Cells stained with epsilon-hemoglobulin showing XX signal will be identified as female fetal cells. Trisomies can be readily identified also based upon whether two or three chromosomes are giving the fluorescence signals.

Alternatively, most of the RBCs can either be carefully lysed using a mild treatment with ammonium chloride lysis reagent (155 mM NH4Cl / 10 mM NaHCO3) to enrich for fetal nucleated red blood cells (fnRBCs) before incubating the sample with a mixture of biotinylated antibodies.

Still another option will be the use of a density gradient such as Ficol 1.073 or Percol 1.073. The buffy coat can then be processed as above to yield fetal nRBCs.

### Example 8

### Detection of Chromosomal Translocations from Captured Circulating Tumor Cells (CTCs) Using Shark and Camelid Heavy Chain Only Antibodies and Their Analogs

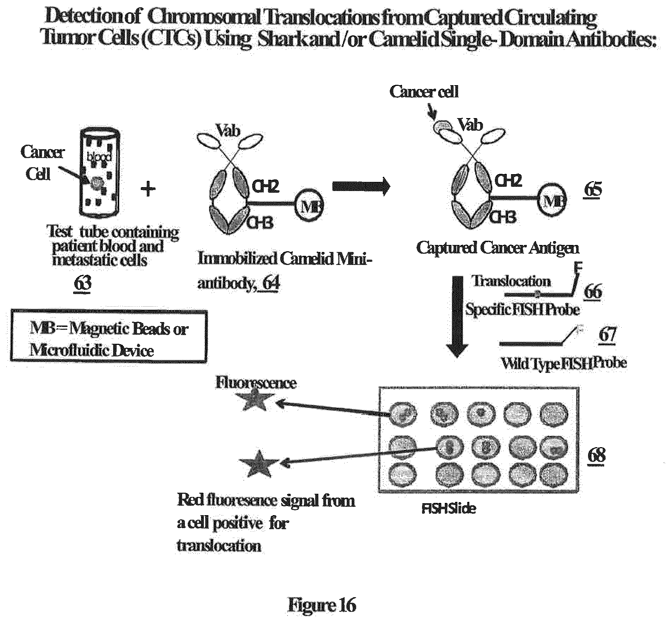

Cells will be captured as described above and also shown in Fig. 16. Enumeration of can be done using an appropriate FISH probes. For example, to test for the presence of TMPRESS2/ERG translocation in case of prostate cancer, FISH probes designed to hybridize with the junction region will be used. Similarly, in case of CML, bcr-Abl FISH probe will be used. An exemplary schematics of the process is shown in Fig. 16.

Unless otherwise defined, all technical and scientific terms used herein have the same meaning as commonly understood by one of ordinary skill in the art to which this invention belongs. All nucleotide sequences provided herein are presented in the 5' to 3' direction.

The inventions illustratively described herein may suitably be practiced in the absence of any element or elements, limitation or limitations, not specifically disclosed herein. Thus, for example, the terms "comprising", "including," containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed.

Thus, it should be understood that although the present invention has been specifically disclosed by preferred embodiments and optional features, modification, improvement and variation of the inventions embodied therein herein disclosed may be resorted to by those skilled in the art, and that such modifications, improvements and variations are considered to be within the scope of this invention. The materials, methods, and examples provided here are representative of preferred embodiments, are exemplary, and are not intended as limitations on the scope of the invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

In addition, where features or aspects of the invention are described in terms of Markush groups, those skilled in the art will recognize that the invention is also thereby described in terms of any individual member or subgroup of members of the Markush group.

All publications, patent applications, patents, and other references mentioned herein are expressly incorporated by reference in their entirety, to the same extent as if each were incorporated by reference individually. In case of conflict, the present specification, including definitions, will control.

Other embodiments are set forth within the following claims.
The present invention also relates to the following items:
1. A method for detecting the presence or absence of an antigen in a sample comprising:
   a) obtaining a sample suspected of having said antigen,
   b) detecting the presence or absence of said antigen in said sample utilizing a polypeptide, wherein said polypeptide comprises all or a portion of at least one variable antigen-binding (Vab) domain of camelid and/ or shark single-domain heavy chain antibodies lacking light-chains, at least ten contiguous amino acids derived from a source other than camelid and/ or shark single-domain heavy chain antibodies lacking light-chains, wherein said polypeptide comprises at least one binding site for an antigen, wherein said polypeptide binds specifically to said antigen, wherein said binding is indicative of the presence of said antigen.
2. The method of item 1, wherein said polypeptide comprises at least two variable antigen-binding (Vab) domains of camelid and/or shark single-domain heavy- chain antibody lacking the light chains.
3. The method of item 2, wherein said two variable antigen-binding (Vab) domains bind to two different antigens.
4. The method of item 1, wherein said polypeptide has three or more variable antigen-binding (Vab) domains of camelid and/or shark single-domain heavy- chain antibody lacking the light chains.
5. The method of item 1, wherein said polypeptide has improved cellular uptake, blood brain barrier permeability, biodistribution and retention.
6. The method of item 1, wherein said polypeptide is immobilized on a solid support prior to binding to said antigen.
7. The method of item 1, wherein said polypeptide binds to said antigen to form a complex, and wherein said complex is immobilized on a solid support.
8. The method of item 1, wherein said polypeptide is linked to at least one entity other than an antibody.
9. The method of item 8, wherein said entity is selected from a group consisting of solid support, radioisotope, enzyme, detectable label, ligand, fluorophore, biotin, digoxegenin, avidin, streptavidin, Fc region of IgGs, a therapeutic agent, toxin, hormone, peptide, protein, vector, siRNA, micro-RNA and nucleic acid.
10. The method of item 8, wherein said solid support is selected from the group consisting of beads, biosensors, nanoparticles, microchannels, microarrays, and microfluidic devices, glass slides, glass chambers, and gold particles.
11. The method of item 8, wherein said enzyme is selected from the group consisting of alkaline phosphatase (AP), horse-raddish-peroxidase (HRP), Luciferase, and beta-galactosidase.
12. The method of item 1, wherein said polypeptide is selected from the group consisting of structures 5, 5a, 7, 7a, 14, 14a, 15, 15a, 19, 20, 31, 32, 33.
13. The method of item 1, wherein said antigen is selected from the group consisting of Aβ42, Tau, ABAD (Abeta-binding alcohol dehydrogenase), a mitochondria regulating protein (MRP), Cyclophilin-D (Cyp-D: MRP), TOM (Translocase of Outer Mitochondria Membrane: MRP), hPReP (Human Presequence Protease: MRP), NMDAR (MRP), PtDS (MRP), mSOD1 (MRP), mHTT (MRP), ApoE4 (Demyelination Regulating Protein: dMRP), integrin-αβ1 (dMRP), integrin-αβ37 (dMRP), PPAR-gamma (dMRP), MAdCAM-1 (dMRP), α-synuclein, TDP-43 (TAR-DNA binding protein-43), ubiquitin, APP, ALZAS, gamma secretase, BACE (β- secretase), Apo-A1, Apo-H, PV-1, PEDF, BDNF, Cystatin C, VGF nerve growth factor inducible, APO-E, GSK-3 binding protein, TEM1, PGD2, EGFR, EGFRT790M, Notch-4, ALDH-1, ESR-1, HER-2/neu, P53, RAS, KLKB1, SMAD4, Smad7, TNF-alfa, HPV, tPA, Mucin, Cadherin-2, FcRn alpha chain, TNF-alfa, Thrombin, cytokerratin 1-20,, Celuloplasmin, Apo AII, VGF, Vif, LEDGF/p75, TS101, gp120, CCR5, CXCR4, HIV protease, HIV integrase, OST-577, H1N1, CD3, CD11a, CD20, CD33, CD25, CD52, Protein C5, VEGF, alfa-4-integrin, EPCA2, PSMA, PSA, TMPRSS2-ERG, PCA3, HAAH, AMACR, Glycoprotein IIb/IIIA, AP-1, VEGF-A, IgG-E, Bacillus anthracis protein, NadD (Nicotinate Mononucleotide Adenyltransferase, a enzyme implicated in drug-resistant bacteria), Plasmodium falciparum, STDs, TB, cGMP directed phosphodiestrase, chain B of Clostridium botulinum neurotroxin type E protein, Borrelia VlsE protein, ACE2 receptor, TTHY, AIAT, AFMN, APOE, SFRS4, SAMP, CD 71, GPA, epsilon-and gamma-glycophorins, TIMP-1, RGIA, EXTL3, biomarkers for: lung cancer, bladder cancer, gastric cancer, brain cancer, breast cancer, prostate cancer, cervical cancer, colorectal cancer, oral cancer, leukemia, childhood neuroblastoma, Non-Hodgkin lymphoma, Alzheimer's disease, Parkinson's disease, and AIDS.
14. A method for diagnosing an individual with a disease, said method comprising:
   a) obtaining a sample from said individual
   b) detecting the presence or absence of one or more biomarkers associated with said disease, wherein said detection comprises utilizing a polypeptide, wherein said polypeptide comprises all or a portion of at least one variable antigen-binding (Vab) domain of camelid and/ or shark single-domain heavy chain antibodies lacking light-chains, at least ten contiguous amino acids derived from a source other than camelid and/ or shark single-domain heavy chain antibodies lacking light-chains, wherein said polypeptide binds specifically to at least one of said biomarkers; and said binding of said polypeptide to one or more of said biomarkers is indicative of the presence of said one or more biomarkers in said sample,
   c) identifying said individual as having said disease when said one or more biomarkers are present in said individual's sample.
15. The method of item 14 further comprising determining the amount of said biomarker in said sample and comparing said amount to a reference value, wherein an amount higher than said reference value is indicative of a disease.
16. The method of item 14, wherein, the said polypeptide is capable of binding specifically to a biomarker selected from the group consisting of:
   biomarkers associated with Alzheimer's Disease, wherein said biomarkers for Alzheimer's disease is selected from the group consisting of Amyloid-beta (Aβ), ALZAS, Tau, Cyclophilinp-D, ABAD, TOM, hPReP, PtDS, PLSCR1, mSOD1, mHTT, integrin-α4β1, integrin-α4β7, PPAR-gamma, MAdCAM-1, NMDAR, integrin- DJ-1, Bax-1, PEDF, HPX, Cystatin-C, Beta-2-Microglobulin, BDNF, Tau-Kinase, gamma-Secretase, beta-Secretase, Apo-E4, and VGF-Peptide;
   biomarkers associated with Parkinson's Disease, wherein said biomarkers for Parkinson's disease is selected from the group consisting of Apo-H, Cerulopasmin, Chromogranin-B, VDBP, Apo-E, Apo-AII, and alfa-Synuclein;
   biomarkers associated with Brain Cancer, wherein said biomarkers for Brain cancer is selected from the group consisting of TEM1, Plasmalemmal Vesicle (PV-1), Prostaglandin D Synthetase, and (PGD-S);
   biomarkers associated with HIV/AIDS, wherein said biomarkers for HIV/AIDS is selected from the group consisting of gp120, Vif, LEDGF/p75, TS101, HIV-Integrase, HIV-Reverse Transcriptase, HIV-Protease, CCR5, and CXCR4;
   biomarkers associated with Lung Cancer, wherein said biomarkers for lung cancer is selected from the group consisting of KRAS, Ki67, EGFR, KLKB1, EpCAM, CYFRA21-1, tPA, ProGRP, Neuron-specific Enolase (NSE), and hnRNP;
   biomarkers associated with Prostate Cancer, wherein said biomarkers for prostate cancer is selected from the group consisting of AMACR, PCA3, TMPRSS2-ERG, HEPSIN, B7-H3, SSeCKs, EPCA-2, PSMA, BAG-1, PSA, MUC6, hK2, PCA-1, PCNA, RKIP, and c-HGK;
   biomarkers associated with Breast Cancer, wherein said biomarkers for breast cancer is selected from the group consisting of EGFR, EGFRT790M, HER-2, Notch-4, ALDH-1, ESR1, SBEM, HSP70, hK-10, MSA, p53, MMP-2, PTEN, Pepsinigen-C, Sigma-S, Topo-11-alfauKPA, BRCA-1, BRCA-2, SCGB2A1, and SCGB1D2;
   biomarkers associated with Colorectal Cancer, wherein said biomarkers for colorectal cancer is selected from the group consisting of SMAD4, EGFR, KRAS, p53, TS, MSI-H, REG1A, EXTL3, p1K3CA, VEGF, HAAH, EpCAM, TEM8, TK1, STAT-3, SMAD-7, beta-Catenin, CK20, MMP-1, MMP-2, MMP-7,9,11, and VEGF-D;
   biomarkers associated with Ovarian Cancer, wherein said biomarkers for ovarian cancer is selected from the group consisting of CD24, CD34, EpCAM, hK8, 10, 13, CKB, Cathesin B, M-CAM, c-ETS1, and EMMPRIN;
   biomarkers associated with Cervical Cancer, wherein said biomarkers for cervical cancer is selected from the group consisting of HPV, CD34, ERCC1, Beta-CF, Id-1, UGF, SCC, p16, p21WAF1, PP-4, and TPS;
   biomarkers associated with Bladder Cancer is selected from the group consisting of CK18, CK20, BLCa-1, BLCA-4, CYFRA21-1, TFT, BTA, Survivin, UCA1, UPII, FAS, and DD23;
   biomarkers associated with a disease causing bacteria, wherein said bacteria or biomarker associated with disease causing bacteria is selected from the group consisting of Clostridium Botulinum, Bacillus Anthracis, Salmonella Typhi, Treponema Pallidum, Plasmodinum, Chlamadyia, Borrelia B, Staphyloccus Aureus, Tetanus, Meningococcal Meningitis, and Mycobacterium tuberculosis, and Nicitinate Mononuceleotide adenyltransferase (NadD);
   biomarkers associated with a disease causing virus, wherein said virus or biomarker associated with a disease causing virus is selected from the group consisting of Pandemic Flu Virus H1N1 strain, Influenza virus H5N1 strain, Hepatitis B virus (HBV) antigen OSt-577, HBV core antigen HBcAg (HBV), HBV antigen Wnt-1, Hepatitis C Virus (HCV) antigen Wnt-1, and HCV RNA.
17. A method for detecting the presence or absence of circulating cells in a sample comprising
   a) obtaining a sample suspected of having circulating cells,
   b) detecting the level of one or more antigen associated with said circulating cell in said sample utilizing a polypeptide, wherein said polypeptide comprises all or a portion of at least one variable antigen-binding (Vab) domain of camelid and/ or shark single-domain heavy chain antibodies lacking light-chains, at least ten contiguous amino acids derived from a source other than camelid and/ or shark single-domain heavy chain antibodies lacking light-chains, wherein said polypeptide binds specifically to said one or more antigens, and wherein said binding of said polypeptide to said antigens is indicative of the presence of circulating cells in said sample.
18. The method of item 17, wherein said circulating cells are circulating tumor cells.
19. The method of item 18, wherein one or more antigens associated with tumor cell is selected from the group consisting of MUC-1, VCAM-1, EpCAm-1, CD44, CD133, E-Cadherin, VEGF, bFGF, sFASL, CD95, p53, Bcl-2 CyclinDl, Cyclin E, TNF-alfa, TGF-beta1, Her-2, EGFR, IGF-1 and IGF-1R, IL-2R, Ras, and cMyc.
20. The method of item 17, wherein said circulating cells are circulating fetal cells.
21. The method of item 20, wherein one or more antigens associated with fetal cell is selected from the group consisting of GPA, CD71, CD 133, CD34, CD44, ITCAM, ITGB1 (Integrin beta-1), Trop-1, Trop-2, HLA-G233, and 6B5.

## Claims

**1.** A micro-antibody analog having the structure: 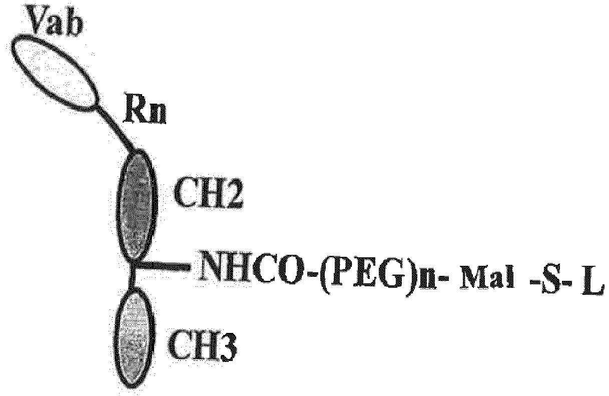 wherein:
(i) Vab is one camelid variable region of a single-domain heavy chain antibody lacking light chains,
(ii) CH2 and CH3 are camelid or human CH2 and CH3 domains,
(iii) Rn represents all of the hinge region of camelid single domain heavy chain antibodies,
(iv) n is 1-50,
(v) Mal is Maleimide derivative, and
(vi) S-L is a thiolated ligand; wherein L is a solid matrix.

**4.** A mini-antibody having the structure: 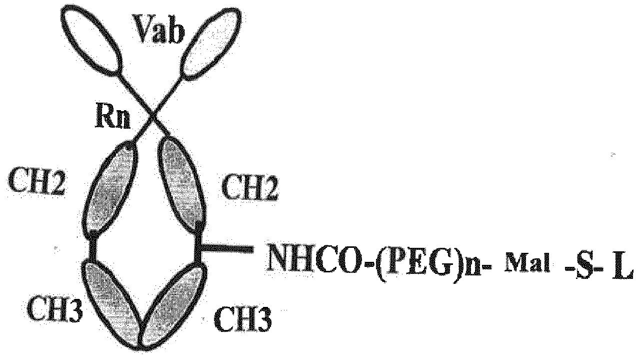 wherein:
(i) Vab is one camelid variable region of a single-domain heavy chain antibody lacking light chains,
(ii) CH2 and CH3 are camelid or human CH2 and CH3 domains,
(iii) Rn represents all of the hinge region of camelid single domain heavy chain antibodies,
(iv) n is 1-50,
(v) Mal is Maleimide, and
(vi) S-L is a thiolated ligand; wherein L is a solid matrix.

**3.** The micro-antibody analog of claim 1 or the mini-antibody analog of claim 2, wherein the solid matrix of (vi) is selected from the group consisting of magnetic beads, glass slides, nanoparticles, microchannels and microfluidic device.

**4.** A diagnostic composition comprising the micro-antibody or mini-antibody analog of any one of claims 1-3.

**5.** A heavy-chain only shark mini-antibody analog having the structure: 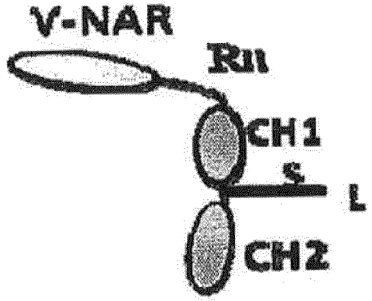 wherein
(i) V-NAR is one shark variable region of a single-domain heavy chain antibody lacking light chains,
(ii) CH1 and CH2 are shark or human CH1 and CH2 domains,
(iii) S is a heterobifunctional linker with one end being capable of conjugating with heavy-chain antibodies and the other end with solid matrixes,
(iv) L is a solid matrix.

**6.** A heavy-chain only shark antibody analog having the structure: 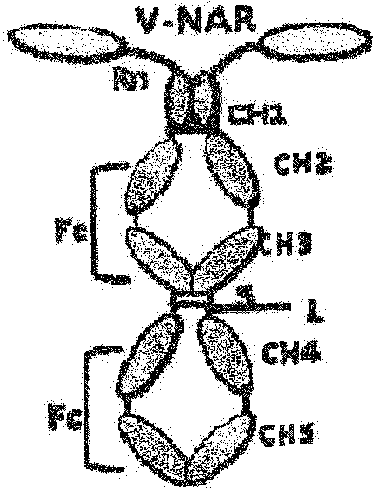 wherein
(i) V-NAR are two shark variable regions of a single-domain heavy chain antibody lacking light chains,
(ii) CH1, CH2 CH3, CH4, and CH5 are shark constant domains,
(iii) S is a heterobifunctional linker with one end being capable of conjugating with heavy-chain antibodies and the other end with solid matrixes, and
(iv) L is a solid matrix.

**7.** The micro-antibody analog or the antibody analog of claims 5 or 6, wherein the solid matrix of (vi) is selected from the group consisting of magnetic beads, glass slides, gold nanoparticles, microchannels and microfluidic device.

**8.** A diagnostic composition comprising the micro-antibody or antibody analog of any one of claims 5-7.
